(19)

**Europäisches Patentamt**

**European Patent Office**

**Office européen des brevets**

(11) **EP 3 892 260 A1**

(12) **EUROPEAN PATENT APPLICATION**

(43) Date of publication:
**13.10.2021 Bulletin 2021/41**

(51) Int Cl.:
*A61K 9/00* [(2006.01)]  *A61K 9/08* [(2006.01)]
*A61K 9/127* [(2006.01)]  *A61K 38/00* [(2006.01)]

(21) Application number: **20169224.1**

(22) Date of filing: **10.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Bayer Animal Health GmbH**
**51373 Leverkusen (DE)**

(72) Inventors:
• **Putzke, Simone**
**50968 Köln (DE)**

• **Thomas, Ilg**
**40789 Monheim (DE)**
• **Elisabeth, Feldhues**
**51465 Bergisch Gladbach (DE)**
• **Iris, Heep**
**50859 Köln (DE)**
• **Lamprecht, Alf**
**50389 Wesseling (DE)**

(74) Representative: **Tier 1 Patents**
**C/o Bayer Intellectual Property GmbH**
**Creative Campus Monheim**
**Bldg. 4685**
**Alfred-Nobel-Straße 10**
**40789 Monheim am Rhein (DE)**

(54) **IMMUNOSTIMULATORY COMPOSITIONS BASED ON LIPOSOMES WITH ZWITERIONIC AND CATIONIC LIPIDS**

(57)    The invention relates to an immunostimulatory composition comprising a liposome-nucleic acid complex wherein said complex contains (1) a liposome which, in terms of its lipids, comprises or consists of a first lipid and a second lipid, wherein the first lipid is a zwitterionic lipid and the second lipid is a cationic lipid, and (2) one or more immunostimulatory oligonucleotides and/or one or more polynucleotides. The invention further relates to certain uses of the immunostimulatory composition and to methods for preparing the same. In particular, the invention concerns the use of a zwitterionic lipid in an immunostimulatory composition comprising a liposome-nucleic acid complex to induce type I interferon immune responses and/or to reduce or even bypass TLR-mediated immune responses.

Figure 1

EP 3 892 260 A1

**Description**

**FIELD OF THE INVENTION**

The invention relates to immunostimulatory compositions comprising certain liposome-nucleic acid complexes, to certain uses thereof and to methods for preparing the same. In particular, the invention concerns the use of a zwitterionic lipid in an immunostimulatory composition comprising a liposome-nucleic acid complex to induce type I interferon immune responses and/or to reduce or even bypass TLR-mediated immune responses.

**BACKGROUND OF THE INVENTION**

The immune systems of vertebrates have evolved defense mechanisms for recognizing invading pathogens. To defeat invading pathogens such as microorganisms, vertebrates have an innate and an adaptive immune system. While the innate immune system forms the first line of defense and permits a rather unspecific immune response against foreign antigens, the adaptive immune system forms the second line of defense and elicits a highly specific immune response. The highly specific adaptive immune response, however, only slowly develops on first exposure to a new pathogen.

The key players in the innate immune systems are phagocytic cells such as dendritic cells, granulocytes or macrophages and several types of innate-like lymphocytes. The response mediated by the innate immune system depends on the recognition of conserved molecular patterns, so-called pathogen-associated molecular patterns (PAMPs), found in many microorganisms. Microbial PAMPs include nucleic acids, lipids and proteins, which are detected by pattern recognition receptors (PRRs) of the innate immune system. Toll-like receptors (TLRs) play a key role as PRRs. TLR4 for example recognizes lipopolysaccharides (LPS) and among the nucleic acid sensing TLRs, including, *inter alia,* TLR3, TLR7, TLR8 and TLR9, which are all localized in endosomal membranes, TLR9 senses unmethylated cytosine-phosphate-guanine (CpG) dinucleotides as danger signals. Upon activation, different PRRs elicit different proinflammatory signaling pathways dependent on, e.g., nuclear factor 'kappa-light-chain-enhancer' of activated B-cells (NF-κB) or interferon regulatory factors (IRF) or both. While activation of NF-κB typically leads to the expression of proinflammatory cytokines, activated IRFs usually cause the production of type I interferon (IFN). Type I interferons (IFNs) are polypeptides that are secreted by infected cells and have three major functions. First, they induce cell-intrinsic antimicrobial states in infected and neighboring cells, thereby limiting the spread of infectious agents. Second, they modulate innate immune responses in a balanced manner that promotes antigen presentation and natural killer cell functions while restraining pro-inflammatory pathways and cytokine production. Third, they activate the adaptive immune system, in particular by promoting the development of high-affinity antigen-specific T and B cell responses and thereby immunological memory.

Unfortunately, pathogen-specific defense mechanisms are not always sufficiently effective as some individuals do not develop acquired resistance until after infection has set in or progressed too far, and in some instances, the pathogen has evolved stealthy means for evading a vertebrate's acquired defenses.

To date, antibiotics are widely used to treat bacterial infectious diseases in humans and animals. In particular large-scale livestock producers, however, are in urgent need of alternatives to antibiotics. There are mainly two reasons for this. Firstly, consumers are increasingly demanding antibiotic-free meat and dairy products and secondly, the occurrence of antibiotic-resistant pathogens has illuminated the dangers of administering antibiotics prophylactically to large populations. One avenue of research aiming to address this problem is to find ways of stimulating the host's immune system so that it can better defeat invading pathogens on its own and/or to develop vaccines with suitable adjuvants. In this regard, there is, however, still a great need for effective immunostimulatory compositions as well as for methods to produce such compositions.

Immunostimulatory compositions comprising nucleic acids that stimulate an innate immune response via immunostimulatory CpG motifs in the nucleic acids have been described (see, e.g., Mui, Barbara, et al. "Immune stimulation by a CpG-containing oligodeoxynucleotide is enhanced when encapsulated and delivered in lipid particles." Journal of Pharmacology and Experimental Therapeutics 298.3 (2001): 1185-1192; Hanagata, Nobutaka. "CpG oligodeoxynucleotide nanomedicines for the prophylaxis or treatment of cancers, infectious diseases, and allergies." International journal of nanomedicine 12 (2017): 515). These primarily act via the TLR9-dependent pathway.

CpG motif-containing DNA has also been used as a vaccine adjuvant to improve the function of professional antigen-presenting cells and boost the generation of humoral and cellular vaccine-specific immune responses (see, e.g., Bode, Christian, et al. "CpG DNA as a vaccine adjuvant." Expert review of vaccines 10.4 (2011): 499-511).

CpG-mediated TLR9 activation, however, can sometimes be particularly undesired, at least if it is the main or only proinflammatory immune response that is stimulated. For example, TLR9 activation has been associated with lung inflammation (Schwartz, David A., et al. "CpG motifs in bacterial DNA cause inflammation in the lower respiratory tract." The Journal of clinical investigation 100.1 (1997): 68-73; Knuefermann, Pascal, et al. "CpG oligonucleotide activates Toll-like receptor 9 and causes lung inflammation in vivo." Respiratory research 8.1 (2007): 72; Ito, Toshihiro, et al. "Toll-like receptor 9 activation is a key mechanism for the maintenance of chronic lung inflammation." American journal of respiratory and critical care medicine 180.12 (2009): 1227-1238). Thus, it is desirable to activate additional or alternative inflammatory pathways apart from classic TLR-mediated responses, such as type I interferon immune response pathways, to induce a potent immune response. The activation of type I interferon immune response pathways can be particularly advantageous, not only for the stimulation of the host's immune system for pathogen defense, but also, e.g., for tumor therapy (see Jablonska, Jadwiga, et al. "Neutrophils responsive to endogenous IFN-β regulate tumor angiogenesis and growth in a mouse tumor model." The Journal of clinical investigation 120.4 (2010): 1151-1164).

The avoidance or suppression of CpG-induced TLR9-mediated inflammatory responses is also desirable in other contexts. For example, in non-viral gene therapy, TLR9-mediated inflammatory responses triggered by the non-viral vectors or the nucleic acids encoding for the desired gene therapy product are a known problem that needs to be avoided (Zhao, Hongmei, et al. "Contribution of Toll-like receptor 9 signaling to the acute inflammatory response to nonviral vectors." Molecular Therapy 9.2 (2004): 241-248.; Makiya Nishikawa, and Yoshinobu Takakura. "Development of safe and effective nonviral gene therapy by eliminating CpG motifs from plasmid DNA vector." Front Biosci 4 (2012): 133-41.). Such inflammatory responses could affect the safety and effectiveness of non-viral gene therapy and in some cases, transgene-expressing cells are eliminated by this response. For this reason, reducing the number of CpG motifs in the constructs has been used to increase the potency of nucleic acid-based gene therapy. In summary, there is a need to avoid TLR-mediated immune responses, in the context of gene therapy or otherwise.

Taken together, in view of the above, there is a need for improved formulations of nucleic acids that activate other immune response pathways, in particular type I interferon pathways, apart from or instead of the classically stimulated TLR-, in particular TLR9-mediated pathways. In the context of gene therapy, there is in particular a need to reduce or even bypass TLR-mediated responses.

## SUMMARY OF THE INVENTION

Against the aforementioned background, it is an object of the present invention to provide an immunostimulatory composition that more efficiently induces cellular inflammatory responses and/or that induces advantageously modified inflammatory responses as compared to compositions known in the art. In particular, it is an object of the present invention to provide an immunostimulatory composition that (also or mainly) activates type I interferon pathways in addition to or instead of TLR-mediated immune responses. A further object of the invention is to provide an immunostimulatory composition that can be produced in an economical manner and is chemically well defined in its components. A yet further object of the invention is to provide a simple and economical process for the production of such compositions. Another object of the invention is to provide an immunostimulatory composition that is effective in preventing or treating an infectious disease in a subject. A further object is to provide an immunostimulatory composition that is useful as a vaccine adjuvant.

Some or all of these objects are achieved by the immunostimulatory composition according to claim 1, the method for preparing the immunostimulatory composition according to claim 11 and the uses according to claims 12 and 13.

The invention provides an immunostimulatory composition comprising a liposome-nucleic acid complex wherein said complex contains

- a liposome which, in terms of its lipids, comprises or consists of a first lipid and a second lipid, wherein the first lipid is a zwitterionic lipid and the second lipid is a cationic lipid; and
- one or more immunostimulatory oligonucleotides and/or one or more polynucleotides.

The inventors have surprisingly found that liposome-nucleic acid complexes, in particular liposome-oligonucleotide complexes, comprising a zwitterionic lipid, in particular 1,2-dioleoyl-*sn*-glycero-3-phosphoethanolamine (DOPE), as one of two compulsory lipid components in the liposomes, are particularly potent and selective in activating cellular type I interferon immune response pathways. Further research into this observation has revealed a possible mechanism of action underlying the invention. Without being bound to any particular scientific theory, the zwitterionic lipid may have special endosomolytic and fusogenic properties that could contribute to this effect. In endosomes, in which liposome-nucleic acid complexes are usually taken up, TLR activation typically takes place. The invention via the use of a zwitterionic lipid in the liposomes seems to make it possible that the immunostimulatory nucleic acids once delivered to the target cells quickly escape from cellular endosomes into the cytosol. This mechanism thus helps to increase the cytosolic concentration of the immunostimulatory nucleic acids. The cytosolic nucleic acids are able to activate IRF-dependent pathways and thus type I interferon immune response pathways. This effect of more pronounced activation of IRF-dependent pathways was observed in particular, when immunostimulatory oligonucleotides were used as nucleic acids in the liposome-nucleic acid complexes of the invention. This was surprising as an interdependency between the type of lipids and the type of nucleic acid has not been previously described.

The second compulsory lipid component in the liposomes of the liposome-nucleic acid complexes is a cationic lipid. Its use in combination with the zwitterionic lipid has two main advantages. On the one hand, the cationic lipid was found to encourage interactions between the lipid bilayer of the liposome and the negatively charged immunostimulatory nucleic acids, allowing for an enrichment of the immunostimulatory nucleic acids in the liposome-nucleic acid complexes. On the other hand, the cationic lipid also functions by providing the liposome with a net positive charge, which in turn facilitates more efficient binding of the liposome-nucleic acid complexes to anionic cell surface molecules, and therefore uptake of the liposome-nucleic acid complexes by the cells.

The invention also provides a method to prepare the immunostimulatory composition of the invention, which comprises the steps of:

A. providing liposomes and mixing the liposomes with one or more immunostimulatory oligonucleotides and/or one or more immunostimulatory polynucleotides to form the liposome-nucleic acid complexes; or

B. contacting lipids with the one or more immunostimulatory oligonucleotides and/or one or more immunostimulatory polynucleotides before or during liposome formation to form liposome-nucleic acid complexes.

In this way, the immunostimulatory composition can be obtained efficiently and in a robust fashion.

The invention provides particularly advantageous results and surprising effects when using immunostimulatory oligonucleotides as the nucleic acid. When using oligonucleotides instead of polynucleotides, e.g. plasmids, in the immunostimulatory compositions, the invention allows a more economical and cleaner production with better characterized reagents while achieving surprisingly similar results in terms of immunostimulation. Immunostimulatory oligonucleotides can be produced by cost-efficient *in vitro* synthesis, while plasmids usually require biotechnological production means such as propagation in bacterial cells, which *per se* results in a more complex, chemically less-well defined product. The ability to synthesize the immunostimulatory oligonucleotides *in vitro* thus has the advantage that a more well-defined and economical chemical product is used to produce the immunostimulatory composition of the invention.

While the immunostimulatory composition and the method of the invention are described in the following mainly in the context of liposome-oligonucleotide complexes, these parts of the description similarly apply to the respective embodiments comprising liposome-polynucleotide complexes.

The present invention also concerns the use of zwitterionic lipid in an immunostimulatory composition comprising a liposome-nucleic acid complex to induce type I interferon immune responses and/or to reduce or bypass TLR-mediated immune responses. These effects are particularly useful not only in therapeutic or prophylactic medical immunostimulation applications, but also in the context of gene therapy since an unwanted immune response against the nucleic acids encoding for the desired gene therapy product can be reduced or even avoided.

Lastly, the invention also concerns the use of the immunostimulatory composition of the invention in a method of stimulating an immune response in a subject and/or a method of preventing or treating an infectious disease in a subject, wherein the method comprises the step of administering the immunostimulatory composition to a subject.

## DETAILED DESCRIPTION OF ILLUSTRATIVE EMBODIMENTS

Features of the immunostimulatory composition, the method and the uses according to the invention that are, for clarity, described herein in the context of separate embodiments, may also be provided in combination in a single embodiment. Conversely, various features of the immunostimulatory composition, the method and the uses according to the inventions that are, for brevity, described in the context of a single embodiment, may also be provided in any subcombination. Similarly, the features or embodiments described in connection with the immunostimulatory composition of the invention are equally applicable to the methods and the uses of the invention and *vice versa.* As used herein, the singular forms "a", "an" and "the" are intended to include the plural forms as well, i.e., in the sense of "one or more", unless the context clearly indicates otherwise.

The immunostimulatory composition

The immunostimulatory composition of the invention comprises a liposome-nucleic acid complex. Said liposome-nucleic acid complex contains a liposome and as nucleic acid one or more immunostimulatory oligonucleotides and/or one ore more immunostimulatory polynucleotides. Preferably the liposome-nucleic acid complex contains a liposome and one or more immunostimulatory oligonucleotides.

An "immunostimulatory composition" as used herein refers to a composition that is capable of activating immune response pathways, in particular that is capable of stimulating the innate immune system of a vertebrate *in vivo.* The term "immune response pathway" is used interchangeably with the terms "inflammatory response pathway" or "proinflammatory response pathway" and refers to intracellular signaling pathways that promote the acute development of an innate immune response. Such immune response pathways include in particular NF-κB dependent pathways that ultimately induce the expression of proinflammatory cytokines and IRF-dependent pathways that trigger the activation of type I interferon immune response pathways.

The immunostimulatory composition is in particular intended for use in a method of stimulating an immune response in a subject and/or a method of preventing or treating an infectious disease in a subject, wherein the method comprises the step of administering the immunostimulatory composition to a subject. Therefore, the immunostimulatory composition may comprise one or more pharmaceutically acceptable carriers and additives.

The immunostimulatory composition of the invention may also comprise one or more antigens and can thus be used as a vaccine formulation, wherein the liposome-nucleic acid complexes serve as adjuvant. Moreover, the immunostimulatory composition of the invention may also comprise one or more drugs. In this case, the immunostimulatory composition is a pharmaceutical composition.

Liposomes

The immunostimulatory composition of the invention comprises liposomes as part of the liposome-nucleic acid complexes. Liposomes as used herein are man-made microscopic particles that are made up of one or more lipid bilayers enclosing a liquid core, which typically is an aqueous liquid core. The liposomes can be unilamellar (in particular large unilamellar vesicles (LUVs), and/or small unilamellar vesicles (SUVs)) or multilamellar vesicles (MLVs). MLVs have multiple bilayers in each vesicle which form several separate compartments. All these types of liposomes have in common that a lipid bilayer encapsulates a liquid core. MLVs, LUVs and SUVs, however, differ in their diameter. MLVs are typically > 500 nm in diameter. LUVs are typically > 100 nm in diameter. SUVs typically have a diameter of about 20 to 100 nm.

Preferably, the liposome is a unilamellar vesicle. Unilamellar vesicles have the advantage that they are generally smaller in diameter than multilamellar liposomes. Due to their smaller size, unilamellar vesciles can be more easily endocytosed by the target cells than the multilamellar liposomes. Moreover, unilamellar vesicles are subjectable to sterile filtration.

In another preferred embodiment of the invention, the liposome is a unilamellar vesicle with an average diameter of between about 10 and about 550 nm. Preferably the liposomes used to form the liposome-nucleic acid complexes of the invention have an average diameter of from about 20 to about 450 nm, even more preferably from about 40 to about 250 nm, still more preferably from 40 to 220 nm and most preferably from 40 to 200 nm. It has been found that the use of liposomes of these size ranges is advantageous since upon complexation of such liposomes with immunostimulatory nucleic acids, liposome-nucleic acid complexes with an appropriate size are formed, which can be efficiently endocytosed by the target cells. An additional advantage of liposome-nucleic acid complexes with an average diameter below about <250 nm is that such liposome-nucleic acid complexes can be sterile-filtered.

The average diameter of liposomes and of liposome-nucleic acid complexes as stated herein are determined by dynamic light scattering, whereby the measurement results are analyzed by the technique of cumulants and the average diameter is calculated as Z-average (see below).

In a most preferred embodiment the liposome is a "small unilamellar vesicle (SUV)". If SUVs are used to form the liposome-nucleic acid complexes of the invention, experiments have shown that the uptake of the liposome-nucleic acid complexes by the target cells is particularly efficient.

The use of the term "about" in connection with numerical values herein indicates that the numerical values may be affected by measurement errors which typically change the numerical values by not more than ± 5%. Numerical values disclosed herein together with the term "about" are also meant to be disclosed as such, i.e. without the term "about". Further, numerical ranges as stated herein are meant to include and disclose each and every value within that range. All upper and lower end points of ranges for the same parameter disclosed herein are combinable with each other. All ranges for different parameters disclosed herein are combinable with each other. In particular, the ranges of the same "preference level" are particularly compatible with each other.

Formation of liposomes requires the presence of "vesicle-forming lipids" which are amphipathic lipids capable of either forming or being incorporated into a bilayer structure. A lipid bilayer includes lipids that are capable of forming a bilayer by themselves or when in combination with another lipid or lipids.

The lipids present in the liposomes according to the invention, i.e. the vesicle-forming lipids, comprise or consist of a first lipid and a second lipid, wherein the first lipid is a zwitterionic lipid and the second lipid is a cationic lipid.

The zwitterionic lipid, i.e. the first lipid that is contained in the liposomes of the invention, contains at a pH of 7 both positive- and negative-charged groups, with an overall neutral net charge.

In a preferred embodiment of the invention, the zwitterionic lipid is a glycerophospholipid wherein two aliphatic moieties are attached to the glycerol moiety by ester and/or ether linkages. The aliphatic moieties can be saturated or unsaturated. The two aliphatic moieties can be the same or different hydrocarbons. Preferably, the two aliphatic moieties of the zwitterionic glycerophospholipid are the same. In another preferred embodiment, the aliphatic moiety is an unbranched unsaturated hydrocarbon containing 10-30, preferably 12-26, more preferably 14-24 and most preferably 16-22 carbon atoms. In a more preferred embodiment, the aliphatic moiety is an unbranched unsaturated hydrocarbon containing 10-30, preferably 12-26, more preferably 14-24 and most preferably 16-22 carbon atoms and a single double bond. In another preferred embodiment of the invention, the charges of the zwitterionic lipid are due to at least a phosphate moiety and a primary amine present in the zwitterionic lipid. The aforementioned preferred embodiments of the zwitterionic lipid can be freely combined with each other. It has been found that the activation of type I interferon immune response pathways is particularly efficient, if the liposomes of the inventive liposome-nucleic acid complexes contain a zwitterionic lipid with any of the aforementioned structural elements.

In the most preferred embodiment of the invention, the zwitterionic lipid is 1,2-dioleoyl-sn-glycero-3-phosphoethanolamine (DOPE). Practical experiments have shown that the use of DOPE together with a cationic lipid in liposomes of liposome- nucleic acid complexes is highly effective to induce type I interferon immune response pathways. These effects were particularly pronounced and specific for the type of zwitterionic lipid used when immunostimulatory oligonucleotides were used as nucleic acid in the nucleic acid-liposome complexes of the invention.

In a preferred embodiment of the invention, the first lipid makes up at least 40 wt.%, preferably at least 45 wt.% and most preferably at least 50 wt.% of the liposome's lipid content. It has been found that the activation of type I interferon immune response pathways is particularly efficient if the zwitterionic lipid is present in these amounts in the lipid bilayer of the liposomes contained in the liposome-nucleic acid complexes of the invention.

The term "second lipid" for the cationic lipid is used to signify that the second lipid is different from the first lipid. In particular, the second lipid does not comprise DOPE. The second lipid is a cationic lipid. The term "cationic lipid" is defined herein as a lipid that has a cationic net charge at any given pH in the range of about 1 to 9, more preferably 2 to 8. The cationic net charge preferably is, but does not have to be present across the entire aforementioned pH range. In another embodiment, cationic lipid is defined as having a cationic net charge at least at a pH of 7.

"Lipid" as used herein preferably means a compound having a molecular weight of less than 3000 Da, more preferably less than 2000 Da.

In a preferred embodiment of the invention, the cationic lipid is selected from the group consisting of N-[1-(2,3-dioleyloxy)propyl]-N,N,N-trimethylammonium chloride (DOTMA), N-[1-(2,3-dioleoyloxy)propyl]-N,N,N-trimethylammonium chloride (DOTAP), dimethyldioctadecylammonium bromide (DDAB), 3β-[N-(N',N'-dimethylaminoethane)-carbamoyl] cholesterol hydrochloride (DC-Cholesterol) and other cationic cholesterol derivatives, N-(4-carboxybenzyl)-N,N-dimethyl-2,3-bis(oleoyloxy)propan-1-aminium (DOBAQ) and 1-[2-(oleoyloxy)ethyl]-2-oleyl-3-(2-hydroxyethyl)imidazolinium chloride (DOTIM).

In a particularly preferred embodiment of the invention, the first lipid is DOPE and the second lipid is DOTMA or the first lipid is DOPE and the second lipid is DOTAP. The research underlying this invention has demonstrated that the use of liposomes with these two kinds of lipid combinations in the immunostimulatory composition are particularly efficient in activating type I interferon immune response pathways.

The charge ratio of cationic lipid to immunostimulatory nucleic acid in the liposome-nucleic acid complexes is preferably 2:1, more preferably of 3:1 and most preferably of 3.5:1. The charge ratio of the liposome-nucleic acid complexes can be assessed by determining the amount of cationic lipid and immunostimulatory nucleic acids that are present in the complexes. Based on the determined amounts, the charge ratio can be calculated. For this calculation, it is assumed that the nucleic acid net charge is derived predominantly from the negatively charged phosphate and/or phosphorothioate groups in the nucleic acid backbone. If these charge ratios are present in the liposome- nucleic acid complexes, particularly reproducible and stable complexes are obtained. Moreover, particularly good loading capacities were observed.

The term "loading capacity" as used herein refers to the capacity of the liposomes to adhere and/or encapsulate all the immunostimulatory nucleic acid.

Immunostimulatory oligonucleotides

The immunostimulatory composition of the invention preferably comprises one or more immunostimulatory oligonucleotides as nucleic acid component. The term "one or more" as used herein means that chemically different oligonucleotides may be complexed with the liposome to form the liposome-oligonucleotide complexes of the immunostimulatory composition. For example, oligonucleotides of different base sequences or differences in the sugar phosphate backbones can be used. In one embodiment, the nucleic acid component of the nucleic acid-liposome complexes of the invention consists of immunostimulatory oligonucleotides.

An "immunostimulatory oligonucleotide" as used herein is an oligonucleotide that elicits an immune response in a vertebrate by being detected as foreign by the vertebrate's innate immune system and thereby activating innate immune response pathways.

Typically, the immunostimulatory oligonucleotides used according to the invention are of synthetic origin. Thus, they can be synthesized with any desired sequence and/or with modifications in the sugar phosphate backbone. Moreover, the immunostimulatory oligonucleotides used in the immunostimulatory composition of the invention have a net negative charge at a pH of 7 and thus represent polyanions. Surprisingly, all the immunostimulatory oligonucleotides tested were able to induce a type I interferon immune response upon complexation with liposomes according to the invention independent of their nucleotide sequence. It therefore is assumed that the technical effect is sequence-independent, the minimal requirement being the polyanionic nature of the immunostimulatory oligonucleotides of the invention.

The one or more immunostimulatory oligonucleotides are linear, at least partially single-stranded DNA molecules. The single-stranded DNA immunostimulatory oligonucleotides may, however, interact with themselves or each other *inter alia* by Watson-Crick base pairing to form secondary structures and agglomerates. Single-stranded stretches will, however, always be present in the immunostimulatory oligonucleotides. In a preferred embodiment of the invention, the one or more immunostimulatory oligonucleotides are CpG oligodesoxynucleotides (or CpG ODN). These are short single-stranded synthetic DNA molecules that contain a cytosine triphosphate deoxynucleotide ("C") followed by a guanine triphosphate deoxynucleotide ("G"). The "p" refers to the phosphodiester link between consecutive nucleotides, although an ODN according to the invention may have a modified phosphorothioate backbone instead. As explained in the introduction, CpG motifs are believed to be recognized as PAMPs by the innate immune system's TLR9 pattern recognition receptor.

In a further preferred embodiment, the immunostimulatory oligonucleotides are selected from the group consisting of A-class, B-class and C-class immunostimulatory oligonucleotides. The classification of immunostimulatory oligonucleotides into "A-class", "B-class" and "C-class" is well-known to the skilled person and described, e.g., in Vollmer, Jörg. "CpG motifs to modulate innate and adaptive immune responses." International reviews of immunology 25.3-4 (2006): 125-134.

A-class immunostimulatory oligonucleotides are typically characterized by a central phosphodiester CpG-containing palindromic motif and a partially phosphorothioate-modified backbone, in particular a phosphorothioate 3' poly-G stretch.

B-class immunostimulatory oligonucleotides are typically characterized by a full phosphorothioate backbone with one or more CpG dinucleotides.

C-class immunostimulatory oligonucleotides exhibit properties of class A and class B immunostimulatory oligonucleotides. They contain a full phosphorothioate backbone and one or more palindromic CpG-containing motif(s).

The immunostimulatory oligonucleotides according to the invention typically have a length of from 14 to 500 nucleotides, preferably from 14 to 400 nucleotides, more preferably from 14 to 300 nucleotides, still more preferably from 14 to 200 nucleotides, even more preferably from 16 to 40 and most preferably from 18 to 30 nucleotides. Immunostimulatory oligonucleotides of this size can be easily synthesized *in vitro* and were found to activate efficiently type I interferon immune response pathways.

In a preferred embodiment of the invention, the one or more immunostimulatory oligonucleotides are selected from the group consisting of B-class and C-class immunostimulatory oligonucleotides. The research underlying the invention demonstrated that with B-class and C-class immunostimulatory oligonucleotides in the inventive immunostimulatory compositions, the activation of type I interferon immune response pathways is particularly efficient.

In another preferred embodiment of the inventive immunostimulatory composition, the one or more immunostimulatory oligonucleotides comprise at least 75% sequence identity, more preferably at least 80% sequence identity, even more preferably 85% sequence identity, still more preferably 90%, 95% or 97% sequence identity with SEQ ID NO. 1, SEQ ID NO.2, SEQ ID NO.3 or SEQ ID NO. 4.

In a more preferred embodiment of the inventive immunostimulatory composition, the one or more immunostimulatory oligonucleotides comprise at least 75% sequence identity, more preferably at least 80% sequence identity, even more preferably 85% sequence identity, still more preferably 90%, 95% or 97% sequence identity with SEQ ID NO.1 or SEQ ID NO.2.

As used herein, "percent sequence identity" and like terms are used to describe the sequence relationships between two or more nucleic acids and are understood in the context of and in conjunction with the terms including: a) reference sequence, b) comparison window, c) sequence identity and d) percentage of sequence identity.

a) A "reference sequence" is a defined sequence used as a basis for sequence comparison. A reference sequence may be a subset of or the entirety of a specified sequence; for example, a segment of a full-length cDNA or gene sequence, or the complete cDNA or gene sequence. In the present case, the reference sequence is SEQ ID NO. 1, SEQ ID NO.2, SEQ ID NO.3 or SEQ ID NO. 4.

b) A "comparison window" includes reference to a contiguous and specified segment of a polynucleotide sequence, wherein the polynucleotide sequence may be compared to a reference sequence and wherein the portion of the polynucleotide sequence in the comparison window may comprise additions, substitutions, or deletions (i.e., gaps) compared to the reference sequence (which does not comprise additions, substitutions, or deletions) for optimal alignment of the two sequences. Those of skill in the art understand that to avoid a misleadingly high similarity to a reference sequence due to inclusion of gaps in the polynucleotide sequence a gap penalty is typically introduced and is subtracted from the number of matches.

c) Methods of alignment of sequences for comparison are well known in the art. Optimal alignment of sequences for comparison may be conducted by the local homology algorithm of Smith and Waterman, Adv. Appl. Math., 2: 482, 1981; by the homology alignment algorithm of Needleman and Wunsch, J. Mol. Biol., 48: 443, 1970; by the search for similarity method of Pearson and Lipman, Proc. Natl. Acad. Sci. USA, 8: 2444, 1988; by computerized implementations of these algorithms, including, but not limited to: CLUSTAL in the PC/Gene program by Intelligenetics, Mountain View, Calif., GAP, BESTFIT, BLAST, FASTA, and TFASTA in the Wisconsin Genetics Software Package, Genetics Computer Group (GCG), 7 Science Dr., Madison, Wis., USA; the CLUSTAL program is well described by Higgins and Sharp, Gene, 73: 237-244, 1988; Corpet, etal., Nucleic Acids Research, 16:881-90, 1988; Huang, et al., Computer Applications in the Biosciences, 8:1-6, 1992; and Pearson, et al., Methods in Molecular Biology, 24:7-331, 1994. The BLAST family of programs which may be used for database similarity searches includes: BLASTN for nucleotide query sequences against nucleotide database sequences; BLASTX for nucleotide query sequences against protein database sequences; TBLASTN for protein query sequences against nucleotide database sequences; and TBLASTX for nucleotide query sequences against nucleotide database sequences. See, Current Protocols in Molecular Biology, Chapter 19, Ausubel, et al., Eds., Greene Publishing and Wiley-Interscience, New York, 1995. New versions of the above programs or new programs altogether will undoubtedly become available in the future, and may be used with the present disclosure.

d) "Percent identity" means the value determined by comparing two optimally aligned sequences over a comparison window, wherein the Portion of the polynucleotide sequence in the comparison window may comprise additions, substitutions, or deletions (i.e., gaps) as compared to the reference sequence (which does not comprise additions, substitutions, or deletions) for optimal alignment of the two sequences. The percentage is calculated by determining the number of positions at which the identical nucleic acid base occurs in both sequences to yield the number of matched positions, dividing the number of matched positions by the total number of positions in the window of comparison and multiplying the result by 100 to yield the percentage of sequence identity.

In one embodiment of the invention, the one or more immunostimulatory oligonucleotides are selected from the group consisting of SEQ ID NO. 1, SEQ ID NO.2, SEQ ID NO.3 and SEQ ID NO. 4. Experimental studies have shown that the use of immunostimulatory oligonucleotides encoding for SEQ ID NO. 1, SEQ ID NO.2, SEQ ID NO.3 and SEQ ID NO.4 in the inventive immunostimulatory composition are particularly efficient in activating type I interferon immune response pathways. The complexation of the immunostimulatory oligonucleotides of SEQ ID NO. 1, SEQ ID NO.2, SEQ ID NO.3 and SEQ ID NO. 4 with the liposomes of the invention transformed said immunostimulatory oligonucleotides from potent TLR-agonists to potent agonists of type I interferon immune response pathways. Without being bound to any particular scientific theory, it is believed that this effect is due to the liposome's lipid composition according to the invention, in particular, the presence of the zwitterionic lipid. This surprisingly seems to result in an effective intracellular release of the immunostimulatory oligonucleotides from endosomes into the cytosol of the target cells.

In a more preferred embodiment of the invention, the one or more immunostimulatory oligonucleotides are oligonucleotides of SEQ ID NO.1 and/or SEQ ID NO.2. Inventive immunostimulatory compositions comprising immunostimulatory oligonucleotides encoding for SEQ ID NO.1 and SEQ ID NO.2 are particularly efficient in activating type I interferon immune response pathways, while at the same time TLR9-mediated immune responses are bypassed.

In a preferred embodiment of the invention, the one or more immunostimulatory oligonucleotides comprise a phosphorothioate in the sugar-phosphate backbone, i.e. a partially phosphorothioate-modified backbone. In another embodiment, the one or more immunostimulatory oligonucleotides comprise a fully phosphorothioate backbone. Phosphorothioate modification has the advantage that the immunostimulatory oligonucleotides are not only protected by their complexation with the liposomes of the invention from degradation by extracellular nucleases, but also by their modified chemical nature. The phosphorothioate modification also protects the immunostimulatory oligonucleotides from intracellular nuclease degradation once they have been taken up into the target cells. As a consequence, the immunostimulatory activity of these immunostimulatory oligonucleotides is increased.

In another preferred embodiment of the invention, the immunostimulatory composition is free of or essentially free of plasmid DNA. "Essentially free" of plasmid DNA means that less than 1 wt%, preferably less than 0.5 wt% and most preferred less than 0.01 wt% of the total DNA content of the immunostimulatory composition is plasmid DNA. By using an essentially plasmid-free or plasmid-free immunostimulatory composition, it is ensured that the inventive immunostimulatory composition only comprises chemically well-defined components. Also, by avoiding the use of plasmids in the immunostimulatory composition, the production costs and complexity of the inventive immunostimulatory composition are reduced.

Immunostimulatory polynucleotides

The immunostimulatory composition of the invention can comprises one or more immunostimulatory polynucleotides. It is also possible, that the immunostimulatory composition of the invention comprises one or more immunostimulatory oligonucleotides and one or more immunostimulatory polynucleotides. In another embodiment, the nucleic acid component of the nucleic acid-liposome complexes of the invention consists of immunostimulatory polynucleotides.

The one or more immunostimulatory polynucleotides can be single-stranded or doublestranded DNA molecules. The immunostimulatory polynucleotides can be circular or linear. In one embodiment, the immunostimulatory polynucleotide is plasmid DNA.

The immunostimulatory polynucleotides of the invention differ from the immunostimulatory oligonucleotides of the invention at least by their length. The immunostimulatory polynucleotides of the invention have a length of at least 31 nucleotides, preferably of at least 41 nucleotides, more preferably of at least 201 nucleotides, still more preferably of at least 301 nucleotides, even more preferably of at least 401 nucleotides and most preferably of at least 501 nucleotides.

Experiments have shown that polynucleotides formulated in the form of the liposome-polynucleotide complexes of the invention, can particularly efficiently activate type I interferon immune response pathways.

Liposome-nucleic acid complexes

The immunostimulatory composition of the invention comprises a liposome-nucleic acid complex. Whereas these are defined herein on the basis of a single complex, it is of course understood that in practice, the immunostimulatory composition of the invention will comprise a plurality of liposome-nucleic acid complexes. These can be of the same or of a different composition. On average, however, the features described herein for the singular liposome-nucleic acid complex and its components, apply to all liposome-nucleic acid complexes in the immunostimulatory composition. Preferably, the liposome-nucleic acid complex is a liposome-oligonucleotide complex.

In a preferred embodiment of the invention, the liposome-nucleic acid complexes at least on average have a diameter of from 20 to 600 nm, preferably from 40 to 500 nm, even more preferably from 60 to 300 nm, still more preferably from 60 to 250 nm and most preferably from 60 to 220 nm. It has been found that the use of complexes of this size range is advantageous since complexes of these size ranges can be endocytosed efficiently by the target cells. An additional advantage of liposome-nucleic acid complexes with an average diameter below about <250 nm is that they can be sterile-filtered.

The skilled person is aware of suitable methods to determine the average diameter of liposomes and liposome-nucleic acid complexes. The average diameters stated herein are Z-Average particle sizes measured by dynamic light scattering (DLS) at a fixed angle of 173° (backscattering mode) with, e.g., a Horiba Nanopartica SZ-100 (HORIBA, Ltd.). When analyzing dynamic light scattering data by the technique of cumulants, the Z-average of the ensemble collection of liposomes or liposome-nucleic acid complexes can be calculated. The translational diffusion coefficient measured by DLS can be converted into Z-Average particle size based on the Stokes-Einstein relation. Z-average size is the intensity weighted harmonic mean size (ISO 22412:2017).

The term "liposome-nucleic acid complex" as used herein, can mean that the immunostimulatory nucleic acids are encapsulated in the liposome and/or that they merely adhere to the surface of the liposome. The complexation of the nucleic acids with the liposome, i.e. their attachment to and/or encapsulation in the liposome has the advantage that the nucleic acids are protected from nuclease degradation. In preferred embodiment of the inventive immunostimulatory composition, the immunostimulatory nucleic acids of the liposome- nucleic acid complexes are at least substantially encapsulated in the liposomes. "Substantially encapsulated" as used herein means, that at least 30%, preferably at least 40%, more preferably at least 50%, even more preferably at least 60% and still more preferably at least 70%, 80%, 90%, 95% or 99% of the immunostimulatory nucleic acids present in the composition are encapsulated by the liposomes. When encapsulated in the liposomes, the immunostimulatory nucleic acids are protected particularly efficiently from degradation, in particular from nucleases. Due to the special lipid composition of the liposomes according to the invention, in particular due to the presumed endosomolytic and fusogenic properties of zwitterionic lipid, the immunostimulatory nucleic acids can exert their immunostimulatory effects very potently even if encapsulated and therefore not directly accessible to the immune system. The invention thus offers the advantage of combining effective protection of the immunostimulatory nucleic acids with robust immunostimulatory effects.

The skilled person knows how to determine the nucleic acid encapsulation degrees. This can be done, e.g., via nuclease protection assays. Here, defined, same-size aliquots of the composition are taken. From a first aliquot, the total amount of nucleic acid present therein is determined by nucleic acid extraction and subsequent determination of the amount of nucleic acids. For a second aliquot, liposome-nucleic acid complexes are incubated with a nuclease that is able to degrade the nucleic acids under conditions that allow for degradation of accessible nucleic acids. Subsequently, the liposomes are washed and the nuclease is inactivated. This is, e.g., achievable by Proteinase K treatment. After extracting the remaining nucleic acids from the nuclease treated liposomes, the amount and integrity of the nucleic acids that survived the nuclease treatment can be analyzed, e.g., by agarose gel electrophoresis. The encapsulation degree can thus be determined by comparing the protected amount of nucleic acids to the total amount of nucleic acids.

The liposome-nucleic acid complexes of the invention have proven to be particularly stable. In one embodiment, the liposome-nucleic acid complexes can be stored in a liquid phase for at least one month, preferably at least three months and most preferably at least six months without loosing their immunostimulatory effect in the cell culture assay described in the Examples herein and/or without changing their properties such as the Z-average, the polydispersity and the Zeta potential.

The polydispersity and the Zeta potential of liposomes can be determined by methods know to the skilled person. The most preferred method to determine the polydispersity is to measure the dynamic light scattering (DLS) at a fixed angle of 173° (backscattering mode) with, e.g., a Horiba Nanopartica SZ-100 (HORIBA, Ltd.) and to derive the polydispersity from the polydispersity index calculated from the cumulants analysis of the DLS-measured intensity autocorrelation function. Assuming a single particle size mode, a single exponential fit is applied to the autocorrelation function and the polydispersity describes the width of the assumed Gaussian distribution. Typically, this parameter is dimensionless and scaled such that values range from 0.05 to 0.7 with small values indicating a monodisperse size distribution ("ISO 22412:2017 - Particle Size Analysis -- Dynamic Light Scattering (DLS)" n.d.). The preferred method to determine the Zeta potential is via electrophoretic light scattering using, e.g., a Horiba Nanopartica SZ-100 (HORIBA, Ltd.). From the known applied electric field and measured particle velocity, the particle mobility can be determined. The Zeta potential is then calculated from the mobility by the Smoluchowski model ("ISO 13099-2:2012 - Colloidal Systems -- Methods for Zeta-Potential Determination -- Part 2: Optical Methods" n.d.).

The skilled person is aware that for immunostimulatory compositions, adjusting the concentration and dose below a given threshold is key for avoiding cytotoxicity. The threshold needs to be determined in dose escalation studies with sample subjects from the desired treatment group. The cytotoxicity can be assessed, e.g., via assays employing 3-(4,5-dimethylthiazol-2-yl)-2,5-diphenyltetrazolium bromide (MTT). The MTT assay is a colorimetric assay for assessing cell metabolic activity which is believed to be proportionally linked to cellular viability. According to the invention, the concentration of the liposome-nucleic acid complex in the immunostimulatory composition is noncytotoxic. "Noncytotoxic" in this context means that the cytotoxicity is below the known or determined threshold of the cytotoxicity measurement method, e.g., the MTT assay. "Cytotoxicity" as used herein, refers to an abnormal cellular state such as failure to thrive, retarded growth, irregular microscopic appearance, and/or decline in immunoresponsiveness. In a preferred embodiment, the concentration of liposome-nucleic acid complex in the the immunostimulatory composition is between about 0.1 and about 250 ng/ml, preferably between about 0.1 and about 200 ng/ml and more preferably between about 0.1 and about 150 ng/ml. In another preferred embodiment of the immunostimulatory composition, the concentration of the immunostimulatory composition is between about 10 and about 250 ng/ml, between about 50 and about 250 ng/ml or between about 100 and about 250 ng/ml.

Method

The invention also provides a method for preparing the immunostimulatory composition described above. The method comprises the steps of:

A. providing liposomes and contacting the liposomes with one or more immunostimulatory oligonucleotides and/or one or more immunostimulatory polynucleotides to form liposome-nucleic acid complexes; or

B. contacting lipids with the one or more immunostimulatory oligonucleotides and/or the one or more immunostimulatory polynucleotides before or during liposome formation to form liposome-nucleic acid complexes.

The lipid component of the liposomes used in step A. and the lipids used in step B. comprise or consist of the first and second lipid as defined above.

Steps A. and B. differ from each other in that either the liposomes are pre-formed before being contacted with the one or more immunostimulatory nucleic acids (step A.) or the liposome formation occurs in the presence of the one or more immunostimulatory nucleic acids (step B.). By preparing the liposome-nucleic acid complexes via step B. a particularly high encapsulation efficiency of the immunostimulatory nucleic acids can be achieved.

Techniques for liposome formation are well known in the art. For example, the chapter on "Liposomal Formulations for Nucleic Acid Delivery" by Ian MacLachlan in "Antisense Drug Technology", CRC Press, 2nd ed. 2007 provides a good overview. For practicing the invention, particular good results were achieved when liposome formation occurred via the thin-film hydration followed by extrusion method (see, e.g., Zhang, Hongwei. "Thin-film hydration followed by extrusion method for liposome preparation." Liposomes. Humana Press, New York, NY, 2017. 17-22; Jahn, Andreas, et al. "Controlled vesicle self-assembly in microfluidic channels with hydrodynamic focusing." Journal of the American Chemical Society 126.9 (2004): 2674-2675; Deamer, David W. "Preparation and properties of ether-injection liposomes." Annals of the New York Academy of Sciences 308.1 (1978): 250-258; Vladisavljevic, Goran T., et al. "Production of liposomes using microengineered membrane and co-flow microfluidic device." Colloids and surfaces A: physicochemical and engineering aspects 458 (2014): 168-177; Kastner, Elisabeth, et al. "High-throughput manufacturing of size-tuned liposomes by a new microfluidics method using enhanced statistical tools for characterization." International journal of pharmaceutics 477.1-2 (2014): 361-368).

In a preferred embodiment of the invention, the liposomes provided in step A. or formed in step B. have one or more features described for the liposomes in the composition of the invention above. This in particular applies to their lamellar type, size, encapsulation degrees and charge ratios.

To produce liposome-nucleic acid complexes that are capable of inducing a response of the innate immune system, a suitable amount of immunostimulatory nucleic acids needs to be added to the provided liposomes in step A. or before or during liposome formation in step B. In a preferred embodiment, the ratio of immunostimulatory nucleic acids to liposomes in step A or in the compositions and complexes of the invention are from 0.1 to 5 $\mu$g of immunostimulatory nucleic acids to about 22 nmol liposomes, preferably from 0.1 to 3 $\mu$g of immunostimulatory nucleic acids to about 22 nmol liposomes and more preferably from 0.1 to 1.5 $\mu$g of immunostimulatory nucleic acids to about 22 nmol liposomes.

### Use of a zwitterionic lipid

The invention further concerns the use of a zwitterionic lipid, in particular DOPE, in an immunostimulatory composition comprising a liposome-nucleic acid complex to induce type I interferon immune responses. In the research that led to the invention, it was found that the use of a zwitterionic lipid in liposomes of liposome-nucleic acid complexes makes it possible to efficiently activate cellular type I interferon immune response pathways. This was surprising, because typically, nucleic acids delivered by liposomes activate TLR-dependent pathways and not cytosolic type I interferon immune response pathways.

It seems that the presence of the zwitterionic lipid, in particular DOPE, in the liposomes accompanying or encapsulating the nucleic acids, changes their immunogenicity and steers the elicited immune response towards the activation of type I interferon immune response pathways. Zwitterionic lipids seem to have special fusogenic properties, which may facilitate intracellular delivery of the complexed nucleic acids by fusion with the membranes of the target cell. Such membrane fusions may occur at a number of different stages of the nucleic acid delivery process such as at the plasma membrane, with endosomal membranes and/or with nuclear membranes. By enabling the fusion with endosomal membranes, the zwitterionic lipid seems to make it possible that the nucleic acids of the liposome-nucleic acid complexes of the invention escape from endosomes into the cytosol of the target cells. Neither the occurrence of this effect nor its utility in altering the elicited immune response by activating type I interferon immune response pathways or the benefits of this alteration in terms of the intended immunostimulation applications was expected or predictable.

In a preferred embodiment of the uses described herein, the zwitterionic lipid has one or more of the characteristics defined for the first lipid contained in the liposome of the invention above.

The skilled person knows suitable *in vitro* cell culture assays for testing whether an immunostimulatory composition activates type I interferon immune response pathways. A particularly suitable way is to use the J774-Dual™ reporter cell line provided by Invivogen (see Examples *infra*). J774-Dual™ cells express the Lucia luciferase gene under the control of the IRF-dependent pathway which ultimately triggers type I interferon immune response pathways. Upon activation, luciferase is transcribed and secreted into the cell culture supernatant. The amount of expressed luciferase can be quantified.

The immunostimulatory compositions of the invention have been found to induce type I interferon immune responses. This may occur in addition to TLR-mediated immune responses. In some embodiments, the immunostimulatory compositions of the invention may next to inducing type I interferon immune responses also reduce or even bypass TLR-mediated immune responses.

Thus, the use of a zwitterionic lipid in an immunostimulatory composition comprising a liposome-nucleic acid complex to induce type I interferon immune responses may, in the target cells, at the same time induce TLR-mediated immune responses or lead to these being reduced or not occurring at all.

Without being bound to any particular scientific theory, it is believed that the use of a zwitterionic lipid also has an effect on TLR-mediated immune response since in particular TLR9 activation takes places in endosomes. The zwitterionic lipid seems to promote the escape of the nucleic acids of the invention to the cytosol. This may be an explanation for the surprising finding that the use of a zwitterionic lipid may reduce or even bypass TLR activation.

The skilled person knows suitable *in vitro* cell culture assays for testing whether an immunostimulatory composition activates cellular TLR-mediated immune response pathways. This again can be done by using the J774-Dual™ reporter cell line provided by Invivogen, which allows for simultaneous assessment of TLR-mediated immune response pathways and IRF-dependent pathways. To enable the assessment of TLR-mediated immune response pathways, J774-Dual™ cells express secreted embryonic alkaline phosphatase (SEAP) under the control of the NF-$\kappa$B pathway which is induced upon TLR activation. SEAP is secreted into the cell culture supernatant. The amount of express SEAP can be easily quantified.

### Medical use

The immunostimulatory compositions described herein are suitable for use in gene therapy, in a method of stimulating an immune response in a subject, and/or a method of preventing or treating an infectious disease in a subject, wherein the method comprises the step of administering the immunostimulatory composition to a subject. The methods comprise administering to a subject an effective amount of the immunostimulatory composition to elicit an immune response. It is also contemplated that more than one administration of the immunostimulatory composition may be used to extend or enhance the immunostimulatory effect.

Upon using the immunostimulatory compositions described herein for gene therapy, in particular with polynucleotides as nucleic acid component of the liposome-nucleic acid complexes, it is possible to reduce or even bypass unwanted TLR-mediated immune responses which are typically induced by the nucleic acids used as gene therapy constructs.

It is believed that stimulating the subject's immune system will allow it to better defeat infectious diseases and invading pathogens on its own. Infectious diseases that seem particularly relevant in the context of the invention are infectious diseases caused by bacteria. It is believed that the compositions and methods of the invention may aid in reducing the amounts of antibiotics required in livestock and meat production.

The immunostimulatory composition of the invention may also be used to enhance the operation of at least one pharmaceutical agent such as a drug or a vaccine, when administered to a subject prior to such agent, co-administered with such agent or administered post administration of such agent. When co-administered with one or more different antigens, the immunostimulatory composition of the invention can be used as an adjuvant in vaccination. Alternatively, when co-administered with one or more different drugs, the immunostimulatory composition of the invention is also a pharmaceutical composition. In this case, the drug molecules are also complexed with the liposomes of the liposome-nucleic acid complexes, i.e. attached to and/or encapsulated into the liposomes. This has the advantage that the elimination half-life of the drug(s) is enhanced and at the same time the delivery of the drug(s) across biological barriers is enhanced.

Furthermore, the liposome-nucleic acid complexes, especially when co-administered with a drug, can be further modified on their surface, e.g. by polymer coating with polyethylene glycol to increase the circulation halftime of the complexes, in particular upon systemic delivery. Moreover, the specificity of the cellular uptake of the inventive liposome-nucleic acid complexes can be further improved by attaching, e.g., antibodies or peptides, to the surface of the complexes for targeting purposes.

Subject as used herein may in particular mean a subject selected from the group consisting of mammal species, aquaculture species, and avian species. The subject may in particular be a non-human animal, which may be selected from the group consisting of cattle, poultry, swine, and companion animals such as dogs and cats.

An effective amount of any of the immunostimulatory compositions described herein may be administered to a subject. The effective amount is sufficient to elicit an immune response in the recipient subject. Such effective amount is any amount that causes an immune response in a recipient subject. Methods of measuring an immune response are well known in the art. Also, a skilled person will recognize that the effective amount will depend upon age weight, stage of infection, as well as other factors known in the art. Suitable effective amounts may range from about 0.01 $\mu$g to 1000 $\mu$g per subject.

Suitable effective amounts may range from about 0.01 $\mu$g to 1,000 $\mu$g per subject. In some embodiments, the effective amount may range from about 0.01 $\mu$g to about 10 $\mu$g, from about 0.01 $\mu$g to about 5 $\mu$g, from about 0.05 $\mu$g to about 5 $\mu$g, from about 0.1 $\mu$g to about 5 $\mu$g, from about 0.05 $\mu$g to about 10 $\mu$g, from about 5 $\mu$g to about 15 $\mu$g, from about 10 $\mu$g to about 15 $\mu$g, from about 10 $\mu$g to about 20 $\mu$g, from about 20 $\mu$g to about 30 $\mu$g, from about 30 $\mu$g to about 40 $\mu$g, from about 40 $\mu$g to about 50 $\mu$g, from about 50 $\mu$g to about 70 $\mu$g, from about 70 $\mu$g to about 90 $\mu$g, from about 50 $\mu$g to about 100 $\mu$g, from about 100 $\mu$g to about 150 $\mu$g, from about 150 $\mu$g to about 200 $\mu$g, from about 200 $\mu$g to about 250 $\mu$g, from about 250 $\mu$g to about 300 $\mu$g, from about 300 $\mu$g to about 350 $\mu$g, from about 350 $\mu$g to about 400 $\mu$g, from about 400 $\mu$g to about 450 $\mu$g, from about 450 $\mu$g to about 500 $\mu$g, from about 500 $\mu$g to about 550 $\mu$g, from about 550 $\mu$g to about 600 $\mu$g, from about 600 $\mu$g to about 650 $\mu$g, from about 650 $\mu$g to about 700 $\mu$g, from about 700 $\mu$g to about 750 $\mu$g, from about 750 $\mu$g to about 800 $\mu$g, from about 800 $\mu$g to about 850 $\mu$g, from about 850 $\mu$g to about 900 $\mu$g, from about 900 $\mu$g to about 950 $\mu$g, from about 950 $\mu$g to about 1000 $\mu$g. Preferably, in some embodiments, the effective amount ranges from about 0.01$\mu$g to about 10 $\mu$g. Yet, preferably in other embodiments the effective amount ranges from about 50 $\mu$g to about 100 $\mu$g. And, preferably in other embodiments, the effective amount ranges from about 40 $\mu$g to about 70 $\mu$g.

In some embodiments, an immune response can be elicited in a member of the avian species by administering an effective amount of any of the immunostimulatory compositions described herein to the member of the avian species. The effective amount is sufficient to elicit an immune response in the member of the avian species. For example, the effective amount of the immunostimulatory composition for an avian species may be from about 0.01 $\mu$g to about 10 $\mu$g, from about 0.05 $\mu$g to about 5 $\mu$g, from about 0.1 $\mu$g to about 1.5 $\mu$g, or from about 1.0 $\mu$g to about 10 $\mu$g. By way of example, suitable effective amounts for a subject that is of the avian species may be about 0.1 $\mu$g, 0.2 $\mu$g, 0.3 $\mu$g, 0.4 $\mu$g, 0.5 $\mu$g, 0.6 $\mu$g, 0.7 $\mu$g, 0.8 $\mu$g, 0.9 $\mu$g, 1.0 $\mu$g, 1.2 $\mu$g, 1.4 $\mu$g, 1.6 $\mu$g, 1.8 $\mu$g, 2.0 $\mu$g, 2.5 $\mu$g, 3.0 $\mu$g, 3.5 $\mu$g, 4.0 $\mu$g, 4.5 $\mu$g, 5.0 $\mu$g, 5.5 $\mu$g, 6.0 $\mu$g, 6.5 $\mu$g, 7.0 $\mu$g, 7.5 $\mu$g, 8.0 $\mu$g, 8.5 $\mu$g, 9.0 $\mu$g, 9.5 $\mu$g, 10.0 $\mu$g, 9.5 $\mu$g, 10.0 $\mu$g,

10.5 $\mu$g, 11.0 $\mu$g, 12 $\mu$g, 13 $\mu$g, 14 $\mu$g, or 15 $\mu$g.

In some embodiments, an immune response can be elicited in a member of the bovine species by administering an effective amount of any of the immunostimulatory compositions described herein to the member of the bovine species. The effective amount is sufficient to elicit an immune response in the member of the bovine species. For example, the effective amount of the immunostimulatory composition for a bovine species can be from about 1 $\mu$g to about 1000 $\mu$g per animal, from about 5 $\mu$g to about 500 $\mu$g per animal, from about 10 $\mu$g to about 100 $\mu$g per animal, from about 10 $\mu$g to about 50 $\mu$g per animal, or from about 40 $\mu$g to about 60 $\mu$g per animal. By way of example, suitable effective amounts for a subject that is of the bovine species may be about 30 $\mu$g, 35 $\mu$g, 40 $\mu$g, 45 $\mu$g, 50 $\mu$g, 55 $\mu$g, 60 $\mu$g, 65 $\mu$g, 70 $\mu$g, 75 $\mu$g, 80 $\mu$g, 85 $\mu$g, 90 $\mu$g, 95 $\mu$g, 100 $\mu$g, 110 $\mu$g, 120 $\mu$g, 130 $\mu$g, 140 $\mu$g, 150 $\mu$g, 160 $\mu$g, 170 $\mu$g, 180 $\mu$g, 190 $\mu$g, 200 $\mu$g or up to 500 $\mu$g, or up to 1000 $\mu$g.

The methods of the invention elicit an immune response in a subject such that the subject is protected from a disease that is amenable to elicitation of an immune response. As used herein, the phrase "protected from a disease" refers to reducing the symptoms of the disease; reducing the occurrence of the disease; reducing the clinical or pathologic severity of the disease; or reducing shedding of a pathogen causing a disease. Protecting a subject can refer to the ability of a composition of the present invention, when administered to a subject, to prevent a disease from occurring, cure, and/or alleviate or reduce disease symptoms, clinical signs, pathology, or causes. For example, without limitation, clinical signs of Bovine Respiratory Disease (BRD) include lung lesions, increased temperature, depression (e.g., anorexia, reduced responsiveness to external stimuli, droopy ears), nasal discharge, and respiratory character (e.g., respiratory rate, respiratory effort). The immunostimulatory compositions described herein may be administered to cattle that are suspected of being exposed to BRD to prevent or reduce the severity of the above-described clinical signs of BRD. By way of further example, without limitation, clinical signs of Marek's disease in avian subjects includes reduced hatchability and bird survivability. The immunostimulatory compositions described herein may be administered to avian subjects that are suspected of being exposed to Marek's disease virus to prevent or reduce the severity of the above-described clinical signs of Marek's disease.

As such, protecting a subject from a disease as used herein encompasses both preventing disease occurrence (prophylactic treatment) and treating a subject that has a disease (therapeutic treatment). In particular, protecting a subject from a disease is accomplished by eliciting an immune response in the subject by inducing a beneficial or protective immune response which may, in some instances, additionally suppress, reduce, inhibit, or block an overactive or harmful immune response. The term "disease" refers to any deviation from the normal health of a subject and includes a state when disease symptoms are present, as well as conditions in which a deviation (e.g., infection, gene mutation, genetic defect, etc.) has occurred, but symptoms are not yet manifested. Methods of the invention may be used for the prevention of disease, stimulation of effector cell immunity against disease, elimination of disease, alleviation of disease, and prevention of a secondary disease resulting from the occurrence of a primary disease.

A variety of administration routes are available for administering the immunostimulatory composition of the invention. The particular mode selected will depend, of course, upon the particular subject group selected, the age and general health status of the subject, the particular condition being treated and the dosage required for therapeutic and/or prophylactic efficacy. The methods of this invention may be practiced using any mode of administration that produces effective levels of an immune response without causing clinically unacceptable adverse effects.

The immunostimulatory composition may be administered intravenously, intramuscularly, intradermally, intraperitoneally, subcutaneously, by spray, in ovo by feather follicle method, orally, intraocularly, intratracheally, intranasally, or by other methods known in the art. In one aspect, the immunostimulatory is administered subcutaneously. In another aspect, the immunostimulatory may be administered intramuscularly. In another aspect, the immunostimulatory is administered as a spray. In another aspect, the immunostimulatory may be administered orally.

As various changes could be made in the above composition, products, and methods without departing from the scope of the invention, it is intended that all matter contained in the above description and in the examples given below shall be interpreted as illustrative and not in a limiting sense.

## SEQUENCE LISTING

This application contains a Sequence Listing which has been submitted electronically and is hereby incorporated by reference in its entirety. Said Sequence Listing file, created April 7, 2020, is named BHC208001.txt and is 1017 bytes in size.

## EXAMPLES

Example 1: Formulating liposomes and nucleic acid complexes

Liposomes were formulated according to the thin-film hydration method followed by two subsequent extrusion cycles. Films were obtained from binary lipid mixtures. A cationic lipid and another lipid were dosed volumetrically from 10 mg/mL stock solution in chloroform into a round-bottom flask. 10 mL chloroform were added and the blend was stirred for 30 minutes at 350 rpm in a water bath adjusted to 37°C (IKA RCT Standard, IKA®-Werke GmbH & Co. KG). Afterwards, the organic solvent was removed using a rotary evaporator to 80 mbar final pressure in a water bath heated to 50°C (Laborata 4000 efficient, Heidolph Instruments). The obtained lipid film was further dried under vacuum at 40°C for three hours (Vacuum drying cabinet VDL series, Binder GmbH). After adding ultrapure water, the emerging suspension was given at least three hours in a tempered water bath under continuous stirring to enable swelling and vesicle budding. It is also possible to perform the vesicle budding, i.e. the liposome formation in the presence of the nucleic acids. For the first extrusion cycle, a Mini Extruder (Avanti Polar Lipids, Inc., USA) was assembled with a polycarbonate membrane (100 nm pore diameter) and the liposome suspension was passed through the filter 13 times. A second extrusion cycle was performed with a 50 nm membrane. Afterwards, the obtained liposomes with a final concentration of 8.7 $\mu$mol/mL per used lipid (17.4 $\mu$mol/mL total lipid) were stored in micro reaction tubes at 4°C.

For complex formulation, 22 nmol liposomes were complexed with 1 $\mu$g nucleic acid (charge ratio cationic lipid/nucleic acid 3.5/1) in a physiological mannitol solution (5.2 wt.% pH 7.2). Equal volumes of the two components were pipetted together and gently mixed for 10 min at 300 rpm and 20°C using Eppendorf Thermomixer® C (Eppendorf, Germany).

Due to high transition temperatures of DDA and DOBAQ from the solid to the liquid crystalline state, the method was slightly modified such that the lipid film was rehydrated in a water bath adjusted to 65°C. Moreover, formulation of DOBAQ-Cholesterol liposomes was not feasible in an equimolar mixture. By adding L-$\alpha$-Phosphatidylcholine, the transition temperature was lowered and cationic liposomes were obtained (DOBAQ/ L-$\alpha$-Phosphatidylcholine/Cholesterol (1:1:2)).

Based on the method described above, 13 cationic liposomes and L-$\alpha$-Phosphatidylcholine/Cholesterol reference liposomes were formulated. The lipid compositions of these liposomes are provided in Table 1. Compositions 1-12 were prepared with 1:1 molar mixtures of the stated first and second lipid. Composition 13 was prepared with a 1:1:2 molar mixture of the three lipids stated.

Table 1: Prepared liposome formulations

|    | First lipid | Second lipid |
|----|-------------|--------------|
| 1  | DOTAP  | Cholesterol (Chol) |
| 2  | DOTAP  | DOPE |
| 3  | DOTAP  | 14:1 L-$\alpha$-Phosphatidylcholine (PC) |
| 4  | DOTMA  | Cholesterol (Chol) |
| 5  | DOTMA  | DOPE |
| 6  | DOTMA  | 14:1 L-$\alpha$-Phosphatidylcholine (14:1 PC) |
| 7  | DC-Chol | Cholesterol (Chol) |
| 8  | DC-Chol | DOPE |
| 9  | DC-Chol | 14:1 L-$\alpha$-Phosphatidylcholine (14:1 PC) |
| 10 | DDA | Cholesterol (Chol) |
| 11 | DDA | DOPE |
| 12 | DDA | 14:1 L-$\alpha$-Phosphatidylcholine (14:1 PC) |
| 13 | DOBAQ | L-$\alpha$-Phosphatidylcholine /Cholesterol (PC/Chol) |

The nucleic acids used for complexation with the liposomes are provided in Table 2.

Table 2: Nucleic acids used for complexation with the liposomes

| Nucleic acid | SEQ ID NO. |
|--------------|------------|
| ODN 2395 | SEQ ID NO. 1 |
| ODN M362 | SEQ ID NO. 2 |
| ODN 1668 | SEQ ID NO. 3 |

(continued)

| Nucleic acid | SEQ ID NO. |
|---|---|
| ODN 2006 | SEQ ID NO. 4 |
| pEX-K248 | |
| pGCMB75.6 | |
| pcDNA3.1(+) | |

Example 2: Liposomes-nucleic acid complex analysis

Liposome-nucleic acid complexes were analyzed in terms of size (Z-Average), polydispersity (PI) and Zeta potential using Horiba Nanopartica SZ-100 (HORIBA, Ltd.). Regarding size and PI, the samples were examined by dynamic light scattering (DLS) at a fixed angle of 173° (backscattering mode) at 25°C in disposable polymethylmetacrylate (PMMA) semi-micro cuvettes. Samples were diluted to 1.08 $\mu$mol/mL and measured five times. The error was calculated as standard deviation (S.D.).

When analyzing DLS data by the use of the technique of cumulants, the Z-Average can be calculated. Since this technique relies on numerically stable least squares fitting, it is relatively insensitive to experimental noise. The translational diffusion coefficient measured by DLS can be converted into Z-Average particle size based on the Stokes-Einstein relation. Z-average size is the intensity weighted harmonic mean size (ISO 22412:2017).

$$D_z = \frac{k_b T}{3\pi\eta D_{t,avg}}$$

$D_z$ = hydrodynamic diameter (particle size)
$D_{t,\,avg}$ = translational diffusion coefficient (by DLS)
$k_B$ = Boltzmann's constant
$T$ = thermodynamic temperature
$\eta$ = dynamic viscosity

The polydispersity is derived from the polydispersity index, which is calculated from the cumulants analysis of the DLS-measured intensity autocorrelation function. Assuming a single particle size mode, a single exponential fit is applied to the autocorrelation function and the polydispersity describes the width of the assumed Gaussian distribution. Typically, this parameter is dimensionless and scaled such that values range from 0.05 to 0.7 with small values indicating a monodisperse size distribution ("ISO 22412:2017 - Particle Size Analysis -- Dynamic Light Scattering (DLS)" n.d.).

The Zeta potential was measured via electrophoretic light scattering using a carbon electrode cell. For this purpose samples were diluted 1:10 in mannitol solution (5.2 (w/v)%, pH 7.2). The error was calculated as standard deviation S.D. of ten independent measurements and the average potential was given in millivolts [mV].

The charge on a particle at the shear plane is designated as Zeta potential. The method of electrophoretic light scattering exploits the fact that a charged particle moves in an applied electric field. The frequency of the scattered light is a function of particle velocity due to the Doppler shift. The measured magnitude of the frequency shift is then used to determine the particle velocity. From the known applied electric field and measured particle velocity, the particle mobility is readily determined. Zeta potential is then calculated from mobility by the Smoluchowski model ("ISO 13099-2:2012 - Colloidal Systems -- Methods for Zeta-Potential Determination -- Part 2: Optical Methods" n.d.).

$$\mu = \frac{\Delta\omega\lambda_0}{4\pi n E \sin\left(\frac{\theta}{2}\right)\sin\left[\left(\frac{\theta}{2}\right) + \zeta\right]}$$

$$\zeta = \frac{3\eta_0\mu}{2\varepsilon f(\kappa a)}$$

$\mu$ = electrophoretic mobility

$\Delta\omega$ = Doppler frequency shift

$\lambda_0$ = laser wavelength

n = medium refractive index

E = electric field change

$\Theta$ = angle between incident and scattered light

$\zeta$ = Zeta potential

$\varepsilon$ = dielectric permittivity

$\eta_0$ = medium viscosity

$f(\kappa a)$ = ratio of the particle radius to electrical double-layer

All calculations were automatically performed by SZ-100 for Windows software.

Example 3: Nucleic acid loading capacity of the liposomes

The nucleic acid loading capacity of the liposome was determined using Nanosep® centrifugal filter devices (Pall Corporation). Liposomes were diluted to 0.11 $\mu$mol/mL and nucleic acids were diluted to 5 or 50 $\mu$g/mL in 1x TE-buffer (10 mM Tris-HCl, 1 mM EDTA, pH 7.5), respectively. Complexes were formulated as described in Example 1.

Liposomes were complexed with 5 $\mu$g/mL nucleic acid. Nanosep® filters were prewashed with TE-buffer and centrifuged for 5 minutes at 14,000 g (Z233 M-2, Hermle AG). 400 $\mu$L of complex were applied separate Nanosep® devices and centrifuged for 5 minutes at 5,000 g. After adding 100 $\mu$L TE-buffer, the tubes were centrifuged for another 20 minutes at 14,000 g. The flow-through was transferred into black 96-well microplates (Perkin Elmer) for fluorescence analysis by either Quant-iT™ OliGreen™ ssDNA assay kit (Thermo Fisher Scientific). All experiments were performed in triplicates.

For ODN 2395 and ODN M362, the loading capacity was determined using Nanosep® 300K centrifugal devices.

As a high affinity to the centrifugal filter membrane was observed in case of ODN M362, filter membranes were passivated prior to complex application. To this end, membranes were saturated with 5% Triton X 100 solution for 1 h and afterwards washed twice with TE-buffer at 14,000 g for 5min. Following such pretreatment steps, the filter units were used for the abovementioned complexes as just described.

Calibration curves for each nucleic acid type were generated using the nucleic acids processes in an analogous manner to their respective lipoplex formulation, i.e. by performing fluorescence analysis by using the Quant-iT™ Oli-Green™ ssDNA assay kit (Thermo Fisher Scientific).

Quant-iT™ OliGreen™ ssDNA assay kit (Thermo Fisher Scientific) was used to fluorometrically quantify ODN concentration in the flowthrough according to the manufacturer's manual. Briefly, 100 $\mu$L Quant-iT™ OliGreen® reagent was added per well, and fluorescence ($\lambda_{ex}$ 485 nm, $\lambda_{em}$ 535 nm) was measured after shaking the plate for 5 min in the dark (Victor 3™ V, Perkin Elmer).

Nucleic acid retrieval rate and loading capacity were calculated through the application of the obtained linear function of the corresponding calibration curve for each nucleic acid type. Dilution factors were adjusted to the linear range of the assay.

Example 4: *In vitro* cell culture

J774-DUAL™ cells (InvivoGen, murine NF-kB & IRF Reporter macrophage-like cells) were grown in Dulbecco's minimum essential medium (DMEM) with high D-glucose content, 1 mM sodium pyruvate and 4 mM stable glutamine. DMEM was supplemented with 10% FBS, 50 $\mu$g/mL streptomycin, 50 U/mL penicillin G and 100 $\mu$g/mL Normocin™. Every other week, the culture medium was additionally supplemented with 200 $\mu$g/mL Zeocin™ and 20 $\mu$g/mL Blasticidin.

For experimental series, serum free DMEM without phenol red was prepared containing 1 mM sodium pyruvate, 4 mM L-glutamine, 50 $\mu$g/mL streptomycin, 50 U/mL penicillin G, 100 $\mu$g/mL Normocin™, 200 $\mu$g/mL Zeocin™ and 20 $\mu$g/mL Blasticidin. J774-DUAL™ cell line was split once per week with a splitting ratio of 1:4.

J774-DUAL™ cells were cultivated in a Heracell™ 150 incubator (Thermo Scientific) at 37°C with 5% CO2 and 95% humidified air. For seeding, cells were counted using a Neubauer bright line hemocytometer (Marienfeld) with a volume 0.1 mm$^3$. Cell lines were regularly checked for absence of mycoplasmic contamination.

Example 5: Immunological readouts in 1774 Dual reporter cell line

J774-Dual™ is a reporter cell line provided by Invivogen, which enables the simultaneous investigation of signal transduction resulting in the activation of the NF-$\kappa$B and/or the IRF pathway.

*NF-κB dependent pathway*

J774-Dual™ cells express secreted embryonic alkaline phosphatase (SEAP) under the control of the NF-κB pathway. SEAP is secreted into the cell culture supernatant. Thus, a semi-quantitative analysis of NF-κB activation is possible (Invivogen product information ("J774-Dual Cells | NF-KB & IRF/IFN Reporter Macrophage" n.d., 774)). p-Nitrophenyl phosphate (pNPP) is a widely utilized chromogenic chemical for rapid determination of alkaline phosphatase activity. In the presence of alkaline phosphatase the substance is hydrolyzed into phosphate and the yellow para-Nitrophenol which can be measured spectrophotometrically (Bessey, Otto A., O. H. Lowky, and Mary Jane Brock. "A method for the rapid determination of alkaline phosphatase with five cubic millimeters of serum." Journal of Biological Chemistry 164 (1946): 321-329.).

Cells were seeded with a density of $1x10^5$ cells per well into 96-well microplates and given 24 h for adherence. The culture medium was removed and the cells were incubated with either naked CpG nucleic acids, liposomes or liposome-nucleic acid complexes in serum-free medium at 37°C with 5% $CO_2$ and 95% humidified air (Heracell™ 150, Thermo Scientific). Liposome with a $LC_{20}$ >40 μM were included in the experimental series. After 48 h of incubation 100 μL supernatant of each sample were transferred into 96-well microplates (SpectraPlate™-96 TC, Perkin Elmer) and analyzed spectrophotometrically using a p-Nitrophenyl phosphate assay.

To this end, 100 μL pNPP working reagent consisting of 20 mM pNPP in 50 mM $NaHCO_3$ buffer (pH 9.6) were added to each well containing samples, controls or calibration standards. After the incubation of the mixture at 37°C for 1 h, absorbance was determined using Victor3 ™ V multimode plate reader (Perkin Elmer) equipped with a 405 nm absorbance filter. Lipopolysaccharide from *Salmonella anterica* serotype Minnesota was applied as calibration standard to determine the linear range of the assay and as positive control. All experiments were conducted in triplicate.

Raw data was normalized to 10 μg/mL protein content and the average value of the untreated control was subtracted.

*Interferon regulatory factor pathway*

J774-Dual™ cells also express the Lucia luciferase gene under the control of the IRF pathway. Upon activation of the latter, luciferase is transcribed and secreted into the cell culture supernatant. By using QUANTI-Luc™ reagent, a semi-quantitative analysis of IRF activation is thus possible (Invivogen product information ("J774-Dual Cells | NF-KB & IRF/IFN Reporter Macrophage" n.d., 774).

Cells were seeded with a density of $1x10^5$ cells per well into 96-well microplates and given 24 h for adherence. The culture medium was removed and the cells were incubated with naked CpG nucleic acids, liposomes or complexes in serum-free medium at 37°C with 5% $CO_2$ and 95% humidified air (Heracell™ 150, Thermo Scientific). Liposome with a $LC_{20}$ >40 μM were included in the experimental series. After 48 h of incubations, 50 μL supernatant of each sample were transferred into 96-well microplates (OptiPlate™-96 HS, Perkin Elmer) and analyzed using QUANTI-Luc™ luminescence assay according to the manufacturer's instructions.

QUANTI-Luc™ reagent was dissolved in 25 mL endotoxin-free water, and 50 μL were added to each well containing samples, controls or calibration standards. Luminescence was immediately determined using EnSpire® multimode plate reader (Perkin Elmer) and measured as relative light units (RLU). Recombinant Lucia luciferase protein was used as calibration standard to determine the linear range of the assay and recombinant IFNα was utilized as positive control. All experiments were conducted in triplicate. Raw data were normalized to 10 μg/mL protein content and the average value of the untreated control was subtracted.

## BRIEF DESCRIPTION OF THE DRAWINGS

The invention will hereinafter be described with reference to the Figures, which depict certain embodiments of the invention. The invention, however, is as defined in the claims and generally described herein. It should not be limited to the embodiments shown for illustrative purposes in the Figures below.

Some of the following figures include significance levels (P<0.05 (*), P<0.01 (**) and P<0.001(***)). These were determined by statistical analysis with Graphpad InStat 3. The comparison of the calculated data with the respective control (and one group with all others, respectively) was conducted using One-way Analysis of Variance (ANOVA) followed by Dunnett Multiple Comparison test. Unpaired t-test with Welch correction was performed for intergroup comparison.

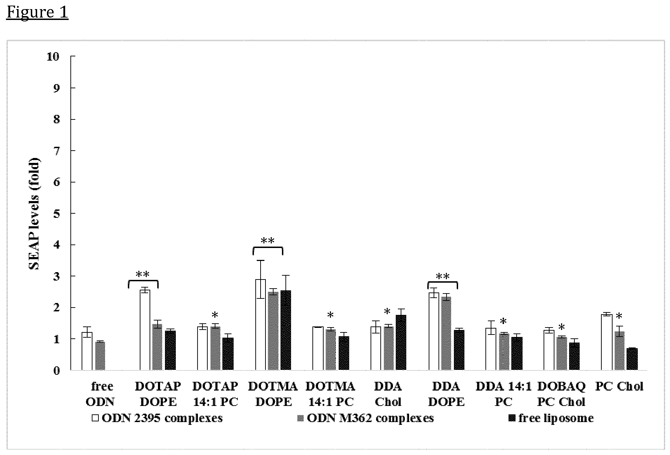

**Figure 1:** Figure 1 shows the activation of the NF-κB pathway by the class C immunostimulatory oligonucleotides ODN 2395 (SEQ ID NO.1) and ODN M362 (SEQ ID NO.2) based on the increase of SEAP levels measured in the supernatant of J774-DUAL™ and the subsequent normalization of the data to the protein content. J774-DUAL™ cells were treated with naked liposomes (i.e. uncomplexed liposomes) with a concentration of 40 μM liposomal

lipids and naked immunostimulatory oligonucleotides (i.e. free or uncomplexed immunostimulatory oligonucleotides) with a concentration of 1.85 μg/mL. For the treatment of J774-DUAL™ cells with oligonucleotide-liposome complexes, the used complexes contained corresponding concentrations of liposomal lipids and immunostimulatory oligonucleotides (i.e. 40 μM liposomal lipids and 1.85 μg/mL immunostimulatory oligonucleotides). Results are given as mean ± SD of three independent experiments. Statistical comparison of the complexes with the respective uncomplexed ODN was not significant unless stated otherwise. Bars which are grouped by a bracket, have the same significance level. Although ODN 2395 (SEQ ID NO.1) and ODN M362 (SEQ ID NO.2) were developed to activate NF-κB immune response pathways (see, e.g., Hartmann, Gunther, and Arthur M. Krieg. "Mechanism and function of a newly identified CpG DNA motif in human primary B cells." The Journal of Immunology 164.2 (2000): 944-953; Marshall, Jason D., et al. "Identification of a novel CpG DNA class and motif that optimally stimulate B cell and plasmacytoid dendritic cell functions." Journal of leukocyte biology 73.6 (2003): 781-792), a potent NF-κB pathway activation was not observed. A stimulation by a factor of 3 can at most be considered as slightly increased immune response compared to the uncomplexed CpG ODN application.

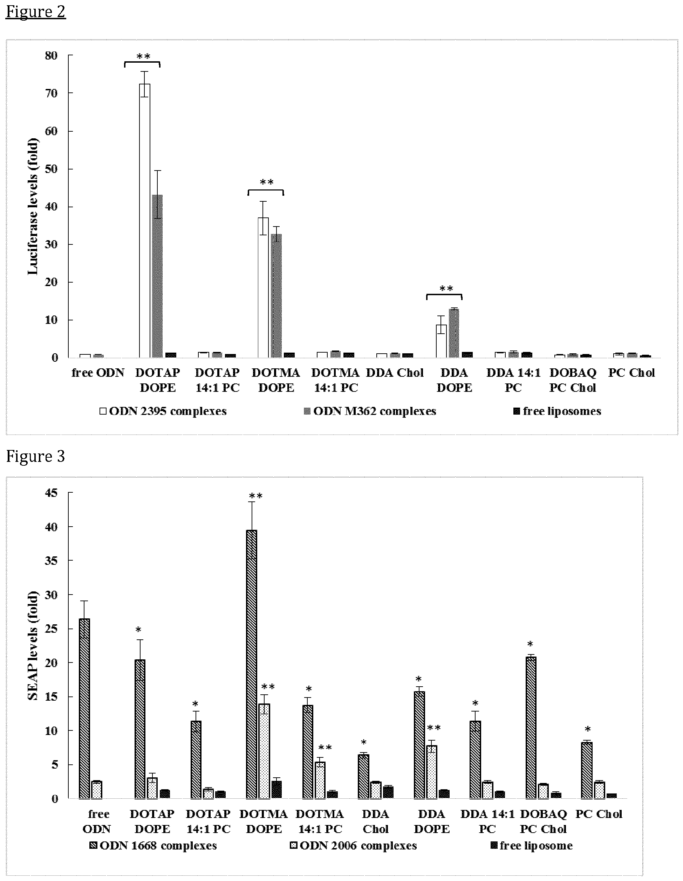

**Figure 2:** Figure 2 shows the activation of the IRF pathway by the class C immunostimulatory oligonucleotides ODN 2395 (SEQ ID NO.1) and ODN M362 (SEQ ID NO.2) based on the increase of luciferase levels measured in the supernatant of J774-DUAL™ and the subsequent normalization of the data to the protein content. J774-DUAL™ cells were treated with naked liposomes with a concentration of 40 μM liposomal lipids and naked immunostimulatory oligonucleotides with a concentration of 1.85 μg/mL. For the treatment of J774-DUAL™ cells with oligonucleotide-liposome complexes, the used complexes contained corresponding concentrations of liposomal lipids and immunostimulatory oligonucleotides. Results are given as mean ± SD of three independent experiments. Statistical comparison of the complexes with the respective uncomplexed ODN was not significant unless stated otherwise. Bars which are grouped by a bracket, have the same significance level. Upon complexation of ODN 2395 (SEQ ID NO.1) and ODN M362 (SEQ ID NO.2) with liposomes comprising the lipids DOTAP + DOPE, DOTMA + DOPE or DDA + DOPE a potent IRF pathway activation was observed.

**Figure 3:** Figure 3 shows the activation of the NF-κB pathway by the class B immunostimulatory oligonucleotides ODN 1668 (SEQ ID NO.3) and ODN 2006 (SEQ ID NO. 4) based on the increase of SEAP levels measured in the supernatant of J774-DUAL™ and the subsequent normalization of the data to the protein content. J774-DUAL™ cells were treated with naked liposomes with a concentration of 40 μM liposomal lipids and naked immunostimulatory oligonucleotides with a concentration of 1.85 μg/mL. For the treatment of J774-DUAL™ cells with oligonucleotide-liposome complexes, the used complexes contained corresponding concentrations of liposomal lipids and immunostimulatory oligonucleotides. Results are given as mean ± SD of three independent experiments. Statistical comparison of the complexes with untreated J774-DUAL™ was not significant unless stated otherwise. The uncomplexed and the complexed ODN 1668 (SEQ ID NO.3) showed an increased NF-κB pathway activation compared to the untreated J774-DUAL™. Except for complexes formed with liposomes comprising the lipids DOTMA + DOPE, the immunostimulatory potency of ODN 1668 (SEQ ID NO.3) was not significantly improved compared to the uncomplexed ODN 1668 (SEQ ID NO. 3). For the ODN 2006 (SEQ ID NO. 4) complexes the NF-κB pathway activation was in most cases not significantly different to the untreated control.

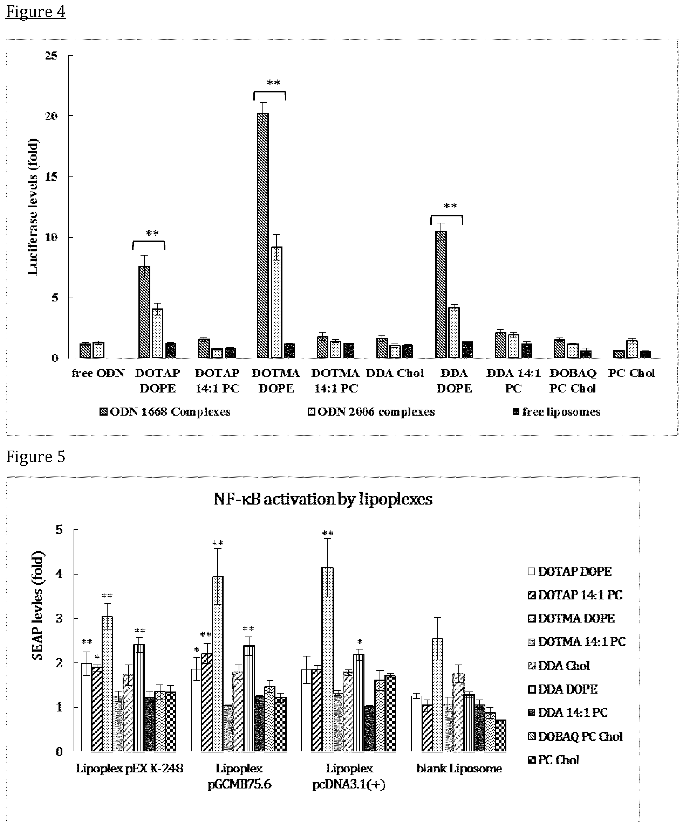

**Figure 4:** Figure 4 shows the activation of the IRF pathway by the class B immunostimulatory oligonucleotides ODN 1668 (SEQ ID NO.3) and ODN 2006 (SEQ ID NO.4) based on the increase of luciferase levels measured in the supernatant of J774-DUAL™ and the subsequent normalization of the data to the protein content. J774-DUAL™ cells were treated with naked liposomes with a concentration of 40 μM liposomal lipids and naked immunostimulatory oligonucleotides with a concentration of 1.85 μg/mL. For the treatment of J774-DUAL™ cells with oligonucleotide-liposome complexes, the used complexes contained corresponding concentrations of liposomal lipids and immunostimulatory oligonucleotides. Results are given as mean ± SD of three independent experiments. Statistical comparison of the complexes with untreated J774-DUAL™ was not significant unless stated otherwise. Bars which are grouped by a bracket, have the same significance level. Upon complexation of ODN 1668 (SEQ ID NO. 3) and ODN 2006 (SEQ ID NO. 4) with liposomes comprising the lipids DOTAP + DOPE, DOTMA + DOPE or DDA + DOPE a potent IRF pathway activation was observed.

**Figure 5:** Figure 5 shows the activation of the NF-κB pathway by the plasmids pEX K-248, pGCMB75.6 and pcDNA3.1(+)based on the increase of SEAP levels measured in the supernatant of J774-DUAL™ and the subsequent normalization of the data to the protein content. J774-DUAL™ cells were treated with naked liposomes with a concentration of 40 μM liposomal lipids and naked plasmids with a concentration of 1.85 μg/mL. For the treatment of J774-DUAL™ cells with plasmid-liposome complexes, the used complexes contained corresponding concentrations of liposomal lipids and plasmids (i.e. 40 μM liposomal lipids and 1.85 μg/mL plasmids). Results are given as mean ± S.D. of three independent experiments. Statistical comparison of the complexes with the PC Chol complexes was not significant unless stated otherwise. A potent NF-κB pathway activation was not observed for the complexes (a maximal amplification factor of about 4 was observed compared to the untreated J774-DUAL™).

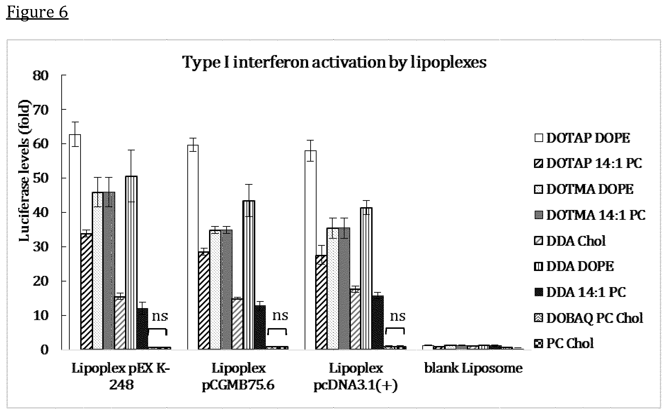

**Figure 6:** Figure 6 shows the activation of the IRF pathway by the plasmids pEX K-248, pGCMB75.6 and pcDNA3.1(+) based on the increase of luciferase levels measured in the supernatant of J774-DUAL™ and the subsequent normalization of the data to the protein content. J774-DUAL™ cells were treated with naked liposomes with a concentration of 40 μM liposomal lipids and naked plasmids with a concentration of 1.85 μg/mL. For the treatment of J774-DUAL™ cells with plasmid-liposome complexes, the used complexes contained corresponding concentrations of liposomal lipids and plasmids. Results are given as mean ± S.D. of three independent experiments. Statistical comparison of the complexes with the PC Chol complexes were significant (P<0.01 (**)) unless stated otherwise. A strong IRF pathway activation was observed for the nucleic acid-liposome complexes except for the complex comprising liposomes containing DOBAQ + PC + Chol or PC + Chol.

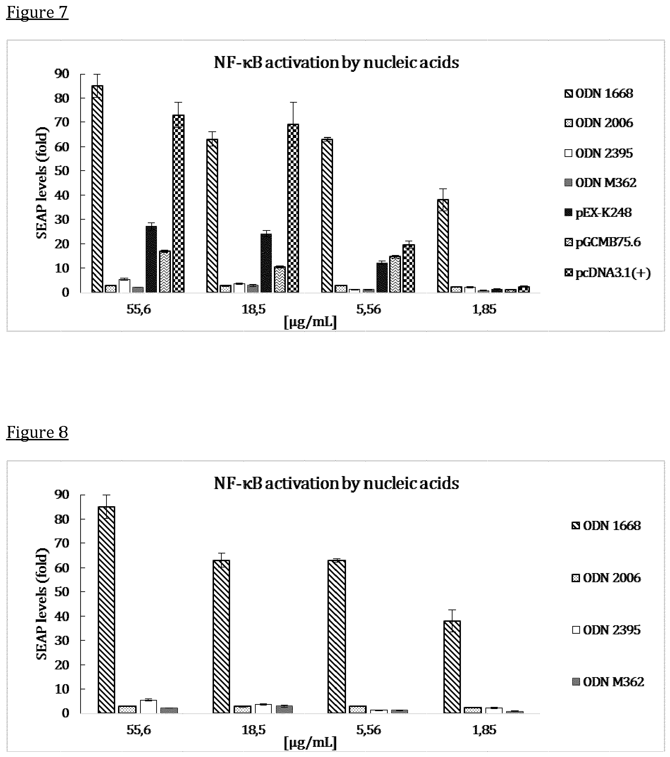

**Figure 7:** Figure 7 shows the activation of the NF-κB pathway by the uncomplexed plasmids pEX K-248, pGCMB75.6 and pcDNA3.1(+) as well as by the uncomplexed ODN 2395 (SEQ ID NO. 1), ODN M362 (SEQ ID NO. 2), ODN 1668 (SEQ ID NO.3) and ODN 2006 (SEQ ID NO.4) based on the increase of SEAP levels measured in the supernatant of J774-DUAL™ and the subsequent normalization of the data to the protein content.

**Figure 8:** Figure 8 shows the activation of the NF-κB pathway by the uncomplexed ODN 2395 (SEQ ID NO. 1), ODN M362 (SEQ ID NO. 2), ODN 1668 (SEQ ID NO.3) and ODN 2006 (SEQ ID NO.4) based on the increase of SEAP levels measured in the supernatant of J774-DUAL™ and the subsequent normalization of the data to the protein content.

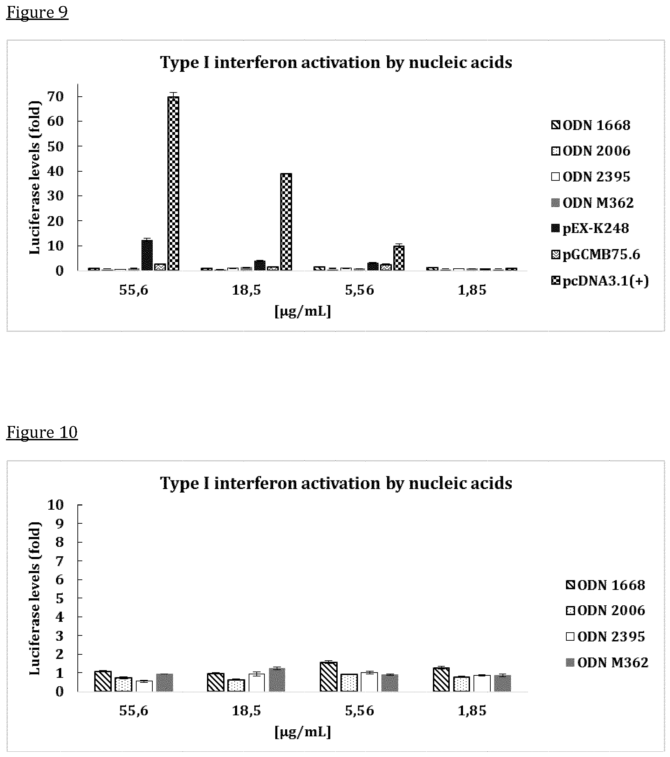

**Figure 9:** Figure 9 shows the activation of the IRF pathway by the uncomplexed plasmids pEX K-248, pGCMB75.6 and pcDNA3.1(+) as well as by the uncomplexed ODN 2395 (SEQ ID NO. 1), ODN M362 (SEQ ID NO. 2), ODN 1668 (SEQ ID NO.3) and ODN 2006 (SEQ ID NO.4) based on the increase of luciferase levels measured in the supernatant of J774-DUAL™ and the subsequent normalization of the data to the protein content.

**Figure 10:** Figure 10 shows the activation of the IRF pathway by the uncomplexed ODN 2395 (SEQ ID NO. 1), ODN M362 (SEQ ID NO. 2), ODN 1668 (SEQ ID NO.3) and ODN 2006 (SEQ ID NO.4) based on the increase of luciferase levels measured in the supernatant of J774-DUAL™ and the subsequent normalization of the data to the protein content.

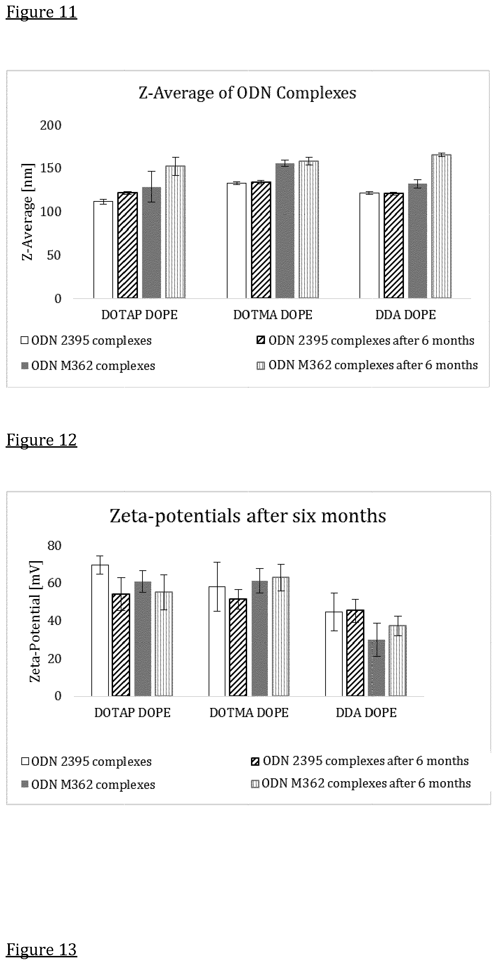

**Figure 11:** Figure 11 shows the Z-average of freshly prepared liposome-oligonucleotide complexes compared to six months old liposome-oligonucleotide complexes comprising as oligonucleotide ODN 2395 (SEQ ID NO.1) or ODN M362 (SEQ ID NO. 2), which was determined as described in Example 2.

**Figure 12:** Figure 12 shows the Zeta-potential of freshly prepared liposome-oligonucleotide complexes compared to six months old liposome-oligonucleotide complexes comprising as oligonucleotide ODN 2395 (SEQ ID NO.1) or ODN M362 (SEQ ID NO.2), which was determined as described in Example 2.

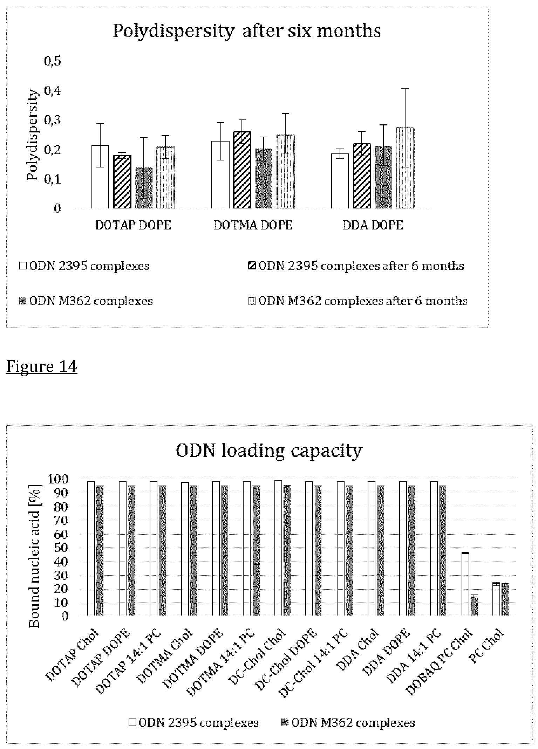

**Figure 13:** Figure 13 shows the polydispersity of freshly prepared liposome-oligonucleotide complexes compared to six months old liposome-oligonucleotide complexes comprising as oligonucleotide ODN 2395 (SEQ ID NO.1) or ODN M362 (SEQ ID NO.2) which was determined as described in Example 2.

**Figure 14:** Figure 14 shows the loading capacity of different liposomal formulations which was determined as described in Example 3.

**Claims**

1. An immunostimulatory composition comprising a liposome-nucleic acid complex wherein said complex contains

   - a liposome which, in terms of its lipids, comprises or consists of a first lipid and a second lipid, wherein the first lipid is a zwitterionic lipid and the second lipid is a cationic lipid; and
   - one or more immunostimulatory oligonucleotides and/or one or more immunostimulatory polynucleotides.

2. The immunostimulatory composition of claim 1, wherein the charges of the zwitterionic lipid are due to at least a phosphate moiety and a primary amine present in the zwitterionic lipid.

3. The immunostimulatory composition of claim 1, wherein the zwitterionic lipid used as the first lipid is 1,2-dioleoyl-sn-glycero-3-phosphoethanolamine (DOPE).

4. The immunostimulatory composition according to any one of the preceding claims, wherein the cationic lipid used as the second lipid is selected from the group consisting of N-[1-(2,3-dioleyloxy)propyl]-N,N,N-trimethylammonium chloride (DOTMA), N-[1-(2,3-dioleoyloxy)propyl]-N,N,N-trimethylammonium chloride (DOTAP), dimethyldioctadecylammonium bromide (DDAB), 3β-[N-(N',N'-dimethylaminoethane)-carbamoyl] cholesterol hydrochloride (DC-Cholesterol) and other cationic cholesterol derivatives, N-(4-carboxybenzyl)-N,N-dimethyl-2,3-bis(oleoyloxy)propan-1-aminium (DOBAQ), 1-[2-(oleoyloxy)ethyl]-2-oleyl-3-(2-hydroxyethyl)imidazolinium chloride (DOTIM), and mixtures thereof.

5. Immunostimulatory composition according to any one of the preceding claims, wherein the first lipid is DOPE and the second lipid is DOTMA or wherein the first lipid is DOPE and the second lipid is DOTAP.

6. Immunostimulatory composition according to any one of the preceding claims, wherein at least 40 wt.%, preferably at least 45 wt.% and most preferably at least 50 wt.%, based on the total weight of lipids in the liposome, is first lipid.

7. The immunostimulatory composition according to any one of the preceding claims wherein the one or more immunostimulatory oligonucleotides are selected from the group consisting of A-class, B-class and C-class immunostimulatory oligonucleotides, and mixtures thereof, and wherein preferably the one or more immunostimulatory oligonucleotides are selected from the group consisting of B-class and C-class immunostimulatory oligonucleotides.

8. The immunostimulatory composition according to any one of the preceding claims, wherein the one or more immunostimulatory oligonucleotides

   (i) comprise at least 75% sequence identity with SEQ ID NO. 1, SEQ ID NO.2, SEQ ID NO.3 or SEQ ID NO. 4, and preferably at least 75% sequence identity with SEQ ID NO.1 or SEQ ID NO.2; or
   (ii) are selected from the group consisting of SEQ ID NO. 1, SEQ ID NO.2, SEQ ID NO.3 and SEQ ID NO. 4, and preferably from the group consisting of SEQ ID NO.1 and SEQ ID NO.2,

9. The immunostimulatory composition according to any one of the preceding claims, wherein at least some phosphodiester moieties in the one or more immunostimulatory oligonucleotides have been chemically modified to increase nuclease resistance, in particular have been replaced by phosphorothioate moieties.

10. The immunostimulatory composition according to any one of the preceding claims, wherein the liposome-oligonucleotide complexes at least on average have a diameter of from 20 to 600 nm, preferably from 40 to 500 nm, even more preferably from 60 to 300 nm, still more preferably from 60 to 250 nm and most preferably from 60 to 220 nm.

11. A method for preparing the immunostimulatory composition according to any one of claims 1 to 10 comprising the steps of:

    A. providing liposomes and contacting the liposomes with one or more immunostimulatory oligonucleotides and/or one or more immunostimulatory polynucleotides to form the liposome-nucleic acid complexes; or
    B. contacting lipids with the one or more immunostimulatory oligonucleotides and/or one ore more immunostimulatory polynucleotides before or during liposome formation to form liposome-nucleic acid complexes.

12. Use of a zwitterionic lipid in an immunostimulatory composition comprising a liposome-nucleic acid complex to induce type I interferon immune responses and/or to reduce or bypass TLR-mediated immune responses.

13. Immunostimulatory composition according to any one of claims 1 to 10 for use in gene therapy, in a method of stimulating an immune response in a subject, and/or a method of preventing or treating an infectious disease in a subject, wherein the method comprises the step of administering the immunostimulatory composition to a subject.

14. Immunostimulatory composition for use according to claim 13 wherein the infectious disease is caused by bacteria.

15. Immunostimulatory composition for use according to claim 13 or 14 wherein the subject is selected from the group consisting of mammal species, aquaculture species, and avian species, in particular wherein the subject is an animal selected from the group consisting of cattle, poultry, swine, and companion animals.

Figure 1

Figure 2

Figure 3

Figure 4

Figure 5

Figure 6

Figure 7

Figure 8

Figure 9

Figure 10

Figure 11

Z-Average of ODN Complexes

□ ODN 2395 complexes      ☑ ODN 2395 complexes after 6 months

▦ ODN M362 complexes      ▥ ODN M362 complexes after 6 months

Figure 12

Zeta-potentials after six months

□ ODN 2395 complexes      ☑ ODN 2395 complexes after 6 months

▦ ODN M362 complexes      ▥ ODN M362 complexes after 6 months

Figure 13

Figure 14

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/10748 A1 (UNIV LONDON PHARMACY [GB]; GREGORIADIS GREGORY [GB]) 19 March 1998 (1998-03-19) | 1-7,9-15 | INV. A61K9/00 A61K9/08 A61K9/127 A61K38/00 |
| Y | * claims 1-32 * <br> * tables 7,8 * <br> * examples 7,8 * | 1-15 | |
| X | WO 01/15726 A2 (INEX PHARMACEUTICALS CORP [CA]; SEMPLE SEAN C [CA] ET AL.) 8 March 2001 (2001-03-08) | 1-7,9-15 | |
| Y | * claims 1-19 * <br> * page 38, line 10 - page 38, line 18 * | 1-15 | |
| X | WO 2016/045732 A1 (BIONTECH RNA PHARMACEUTICALS GMBH [DE]) 31 March 2016 (2016-03-31) | 1-7,9-15 | |
| Y | * example 5 * <br> * example 6 * <br> * claims 1-33 * <br> * figures 1-5 * | 1-15 | |

TECHNICAL FIELDS
SEARCHED (IPC)

A61K

The present search report has been drawn up for all claims

| Place of search | Date of completion of the search | Examiner |
|---|---|---|
| Munich | 22 October 2020 | Schifferer, Hermann |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 16 9224

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2020

| Patent document cited in search report | Publication date | Patent family member(s) | | Publication date |
|---|---|---|---|---|
| WO 9810748 A1 | 19-03-1998 | AT | 228824 T | 15-12-2002 |
| | | AT | 395904 T | 15-06-2008 |
| | | AU | 728581 B2 | 11-01-2001 |
| | | CA | 2271388 A1 | 19-03-1998 |
| | | CN | 1237102 A | 01-12-1999 |
| | | DE | 69717661 T2 | 25-09-2003 |
| | | EP | 0938298 A1 | 01-09-1999 |
| | | EP | 1254657 A2 | 06-11-2002 |
| | | ES | 2187812 T3 | 16-06-2003 |
| | | ES | 2305157 T3 | 01-11-2008 |
| | | JP | 2001502299 A | 20-02-2001 |
| | | KR | 20000036088 A | 26-06-2000 |
| | | US | 2008286353 A1 | 20-11-2008 |
| | | WO | 9810748 A1 | 19-03-1998 |
| WO 0115726 A2 | 08-03-2001 | AT | 376838 T | 15-11-2007 |
| | | AU | 780535 B2 | 24-03-2005 |
| | | BR | 0013834 A | 23-04-2002 |
| | | CA | 2382611 A1 | 08-03-2001 |
| | | CN | 1501811 A | 02-06-2004 |
| | | CZ | 20021029 A3 | 14-08-2002 |
| | | DE | 60036950 T2 | 07-08-2008 |
| | | EP | 1212085 A2 | 12-06-2002 |
| | | HU | 0202327 A2 | 28-10-2002 |
| | | JP | 2003509341 A | 11-03-2003 |
| | | KR | 20020068500 A | 27-08-2002 |
| | | MX | PA02002107 A | 20-08-2003 |
| | | PL | 354879 A1 | 08-03-2004 |
| | | WO | 0115726 A2 | 08-03-2001 |
| WO 2016045732 A1 | 31-03-2016 | AU | 2015320980 A1 | 06-04-2017 |
| | | BR | 112017005821 A2 | 12-12-2017 |
| | | CA | 2960934 A1 | 31-03-2016 |
| | | CN | 107072945 A | 18-08-2017 |
| | | EP | 3197433 A1 | 02-08-2017 |
| | | JP | 6703982 B2 | 03-06-2020 |
| | | JP | 2017532322 A | 02-11-2017 |
| | | KR | 20170063780 A | 08-06-2017 |
| | | RU | 2017113970 A | 25-10-2018 |
| | | SG | 11201702182Y A | 27-04-2017 |
| | | US | 2017273907 A1 | 28-09-2017 |
| | | WO | 2016045732 A1 | 31-03-2016 |
| | | WO | 2016046060 A1 | 31-03-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MUI, BARBARA et al.** Immune stimulation by a CpG-containing oligodeoxynucleotide is enhanced when encapsulated and delivered in lipid particles. *Journal of Pharmacology and Experimental Therapeutics,* 2001, vol. 298.3, 1185-1192
- **HANAGATA ; NOBUTAKA.** CpG oligodeoxynucleotide nanomedicines for the prophylaxis or treatment of cancers, infectious diseases, and allergies. *International journal of nanomedicine,* 2017, vol. 12, 515
- **BODE ; CHRISTIAN et al.** CpG DNA as a vaccine adjuvant. *Expert review of vaccines,* 2011, vol. 10.4, 499-511
- **SCHWARTZ ; DAVID A. et al.** CpG motifs in bacterial DNA cause inflammation in the lower respiratory tract. *The Journal of clinical investigation,* 1997, vol. 100.1, 68-73
- **KNUEFERMANN ; PASCAL et al.** CpG oligonucleotide activates Toll-like receptor 9 and causes lung inflammation in vivo. *Respiratory research,* 2007, vol. 8.1, 72
- **ITO ; TOSHIHIRO et al.** Toll-like receptor 9 activation is a key mechanism for the maintenance of chronic lung inflammation. *American journal of respiratory and critical care medicine,* 2009, vol. 180.12, 1227-1238
- **JABLONSKA ; JADWIGA et al.** Neutrophils responsive to endogenous IFN-β regulate tumor angiogenesis and growth in a mouse tumor model. *The Journal of clinical investigation,* 2010, vol. 120.4, 1151-1164
- **ZHAO ; HONGMEI et al.** Contribution of Toll-like receptor 9 signaling to the acute inflammatory response to nonviral vectors. *Molecular Therapy,* 2004, vol. 9.2, 241-248
- **MAKIYA NISHIKAWA ; YOSHINOBU TAKAKURA.** Development of safe and effective nonviral gene therapy by eliminating CpG motifs from plasmid DNA vector. *Front Biosci,* 2012, vol. 4, 133-41
- **VOLLMER, JÖRG.** CpG motifs to modulate innate and adaptive immune responses. *International reviews of immunology,* 2006, vol. 25.3-4, 125-134
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 8, 2444

- **HIGGINS ; SHARP.** *Gene,* 1988, vol. 73, 237-244
- **CORPET et al.** *Nucleic Acids Research,* 1988, vol. 16, 881-90
- **HUANG et al.** *Computer Applications in the Biosciences,* 1992, vol. 8, 1-6
- **PEARSON et al.** *Methods in Molecular Biology,* 1994, vol. 24, 7-331
- Current Protocols in Molecular Biology. Greene Publishing and Wiley-Interscience, 1995
- Liposomal Formulations for Nucleic Acid Delivery. **IAN MACLACHLAN.** Antisense Drug Technology. CRC Press, 2007
- Thin-film hydration followed by extrusion method for liposome preparation. **ZHANG, HONGWEI.** Liposomes. Humana Press, 2017, 17-22
- Controlled vesicle self-assembly in microfluidic channels with hydrodynamic focusing. **JAHN ; ANDREAS et al.** Journal of the American Chemical Society. 2004, vol. 126.9, 2674-2675
- **DEAMER ; DAVID W.** Preparation and properties of ether-injection liposomes. *Annals of the New York Academy of Sciences,* 1978, vol. 308.1, 250-258
- **VLADISAVLJEVIC ; GORAN T. et al.** Production of liposomes using microengineered membrane and co-flow microfluidic device. *Colloids and surfaces A: physicochemical and engineering aspects,* 2014, vol. 458, 168-177
- **KASTNER ; ELISABETH et al.** High-throughput manufacturing of size-tuned liposomes by a new microfluidics method using enhanced statistical tools for characterization. *International journal of pharmaceutics,* 2014, vol. 477.1-2, 361-368
- **BESSEY, OTTO A. ; O. H. LOWKY ; MARY JANE BROCK.** A method for the rapid determination of alkaline phosphatase with five cubic millimeters of serum. *Journal of Biological Chemistry,* 1946, vol. 164, 321-329
- **HARTMANN, GUNTHER ; ARTHUR M. KRIEG.** Mechanism and function of a newly identified CpG DNA motif in human primary B cells. *The Journal of Immunology,* 2000, vol. 164.2, 944-953
- **MARSHALL ; JASON D. et al.** Identification of a novel CpG DNA class and motif that optimally stimulate B cell and plasmacytoid dendritic cell functions. *Journal of leukocyte biology,* 2003, vol. 73.6, 781-792

SEQUENCE LISTING

<110> BAYER ANIMAL HEALTH GMBH

<120> Immunostimulatory Compositions

<130> BHC208001

<160> 4

<170> BiSSAP 1.3.6

<210> 1
<211> 22
<212> DNA
<213> Artificial Sequence


<220>
<223> ODN 2395 is an immunostimulatory oligonucleotide.

<400> 1
tcgtcgtttt cggcgcgcgc cg                                          22


<210> 2
<211> 25
<212> DNA
<213> Artificial Sequence


<220>
<223> ODN M362 is an immunostimulatory oligonucleotide.

<400> 2
tcgtcgtcgt tcgaacgacg ttgat                                      25


<210> 3
<211> 20
<212> DNA
<213> Artificial Sequence


<220>
<223> ODN 1668 is an immunostimulatory oligonucleotide.

<400> 3
tccatgacgt tcctgatgct                                            20


<210> 4
<211> 24
<212> DNA
<213> Artificial Sequence


<220>
<223> ODN 2006 is an immunostimulatory oligonucleotide.

<400> 4
tcgtcgtttt gtcgttttgt cgtt                                       24